# EUROPEAN PATENT APPLICATION

(11) **EP 4 353 705 A1**
(43) Date of publication of application: **17.04.2024**
(21) Application number: 22807291.4
(22) Date of filing: 11.04.2022
(51) Int. Cl.: C07C 29/147, C07C 31/38, C07C 31/42, C07C 41/26, C07C 43/178, C07C 45/64, C07C 49/173

(54) **ALCOHOL PRODUCTION METHOD AND COMPOSITION**

(30) Priority: 10.05.2021 JP 2021079979
(71) Applicant: DAIKIN INDUSTRIES, LTD., Osaka-Shi, Osaka 530-0001 (JP)
(72) Inventor: SUZUKI, Yuuki, Osaka-Shi, Osaka 530-0001 (JP); OGATA, Akitoshi, Osaka-Shi, Osaka 530-0001 (JP); YOSHIYAMA, Asako, Osaka-Shi, Osaka 530-0001 (JP); MATSUURA, Makoto, Osaka-Shi, Osaka 530-0001 (JP); KUROKI, Yoshichika, Osaka-Shi, Osaka 530-0001 (JP); KISHIKAWA, Yosuke, Osaka-Shi, Osaka 530-0001 (JP)
(74) Representative: Hoffmann Eitle
(86) International application number: PCT/JP2022/017520
(87) International publication number: WO 2022/239587

(57) **Abstract**

The disclosure aims to provide an alcohol production method that can successfully reduce the amount of solvents used, reduce the environmental load and cost, and produce an alcohol at high concentration and in high yield. The alcohol production method includes a step (1) of reacting a compound (1) represented by the formula (1) and a metal hydride compound under conditions where at least one solvent selected from the group consisting of water and a C3 or higher protic solvent is present and the compound (1) is at a concentration of 1.0 mmol/g (amount of substance of compound (1)/total solvent amount) or higher to provide a compound (2) represented by the formula (2).

## Description

### TECHNICAL FIELD

The disclosure relates to alcohol production methods. The disclosure also relates to alcohol-containing compositions.

### BACKGROUND ART

Known alcohol production methods include synthesis by reduction of esters using metal hydride compounds such as boron hydride compounds and aluminum hydride compounds (for example, see Patent Literatures 1 to 5).

### CITATION LIST

### - Patent Literature

Patent Literature 1: JP 2016-20340 A
Patent Literature 2: WO 2007/058852
Patent Literature 3: JP 2014-167596 A
Patent Literature 4: US 5969074 B
Patent Literature 5: JP 2018-90492 A

### SUMMARY OF INVENTION

### - Technical Problem

The disclosure aims to provide an alcohol production method that can successfully reduce the amount of solvents used, reduce the environmental load and cost, and produce an alcohol at high concentration and in high yield.

### - Solution to Problem

The disclosure relates to an alcohol production method including:
a step (1) of reacting a compound (1) and a metal hydride compound under conditions where at least one solvent selected from the group consisting of water and a C3 or higher protic solvent is present and the compound (1) is at a concentration of 1.0 mmol/g (amount of substance of compound (1)/total solvent amount) or higher to provide a compound (2),
the compound (1) being represented by the following formula (1): 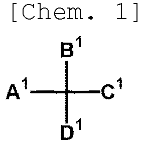 wherein
   A¹ to D¹ are each independently
      hydrogen,
      fluorine,
      a hydroxy group,
      a C1-C10 hydrocarbon group in which at least one hydrogen atom is optionally replaced by a fluorine atom and which optionally contains a cyclic structure, a multiple bond, a carbonyl group, or an ether bond in a structure,
      a substituent represented by the following formula (A): 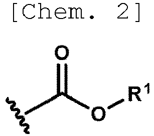
   wherein R¹ is hydrogen or a C1-C10 hydrocarbon group in which at least one hydrogen atom is optionally replaced by a fluorine atom and which optionally contains a cyclic structure, a multiple bond, a carbonyl group, or an ether bond in a structure, or
      a substituent represented by the following formula (B): 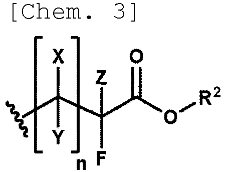
   wherein
      n is 0 to 5;
      X, Y, and Z are each independently
         hydrogen,
         fluorine, or
         a C1-C10 hydrocarbon group in which at least one hydrogen atom is optionally replaced by a fluorine atom and which optionally contains a cyclic structure, a multiple bond, a carbonyl group, or an ether bond in a structure; and
      R² is hydrogen or a C1-C10 hydrocarbon group in which at least one hydrogen atom is optionally replaced by a fluorine atom and which optionally contains a cyclic structure, a multiple bond, a carbonyl group, or an ether bond in a structure,
   not all of A¹ to D¹ are substituents represented by the formula (A),
   at least one selected from A¹ to D¹ is a substituent represented by the formula (A) or the formula (B),
   in the case where one to three selected from A¹ to D¹ are substituents represented by the formula (A), one or more of remaining substituents are fluorine, and
   in the case where two or three selected from A¹ to D¹ are substituents each represented by the formula (A) or the formula (B), R¹ and R² are optionally bonded to each other at one or more carbon atoms each at any position,
   the compound (2) being represented by the following formula (2): 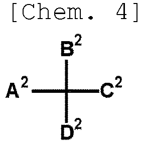 wherein
      A² to D² are each independently
      hydrogen,
      fluorine,
      a hydroxy group,
      a C1-C10 hydrocarbon group in which at least one hydrogen atom is optionally replaced by a fluorine atom and which optionally contains a cyclic structure, a multiple bond, a carbonyl group, or an ether bond in a structure,
      a substituent represented by the following formula (C) : 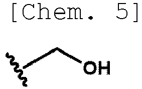 , or
      a substituent represented by the following formula (D) : 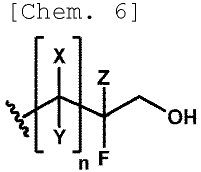
      wherein
         n is 0 to 5; and
         X, Y, and Z are each independently
            hydrogen,
            fluorine, or
            a C1-C10 hydrocarbon group in which at least one hydrogen atom is optionally replaced by a fluorine atom and which optionally contains a cyclic structure, a multiple bond, a carbonyl group, or an ether bond in a structure,
      not all of A² to D² are substituents represented by the formula (C),
      at least one selected from A² to D² is a substituent represented by the formula (C) or the formula (D), and
      in the case where one to three selected from A² to D² are substituents represented by the formula (C), one or more of remaining substituents are fluorine.

The C3 or higher protic solvent preferably includes at least one selected from the group consisting of n-propyl alcohol, isopropyl alcohol, n-butyl alcohol, sec-butyl alcohol, isobutyl alcohol, tert-butyl alcohol, n-pentyl alcohol, sec-pentyl alcohol, isopentyl alcohol, and neopentyl alcohol.

The metal hydride compound preferably includes at least one selected from the group consisting of a boron hydride compound and an aluminum hydride compound, more preferably at least one selected from the group consisting of lithium borohydride, lithium aminoborohydride, lithium triethylborohydride, lithium tri(sec-butyl)borohydride, potassium borohydride, potassium tri(sec-butyl)borohydride, calcium borohydride, sodium borohydride, sodium cyanoborohydride, sodium triacetoxyborohydride, sodium bis(2-methoxyethoxy)aluminum hydride, zinc borohydride, nickel borohydride, and diborane, still more preferably at least one selected from the group consisting of sodium borohydride, sodium cyanoborohydride, and sodium triacetoxyborohydride.

The metal hydride compound is preferably used in an amount of 1.2 equivalents or less relative to the ester group in the compound (1).

Preferably, X, Y, and Z are each independently hydrogen, fluorine, or a C1-C5 hydrocarbon group in which at least one hydrogen atom is optionally replaced by a fluorine atom.

Preferably, n is 0 to 3 and X, Y, and Z are each independently hydrogen or fluorine.

Preferably, R¹ or R² is a methyl group, an ethyl group, a n-propyl group, an isopropyl group, a n-butyl group, a sec-butyl group, an isobutyl group, a tert-butyl group, a n-pentyl group, a sec-pentyl group, an isopentyl group, a neopentyl group, a hexyl group, a 2-methylpentyl group, or a heptyl group in which at least one hydrogen atom is optionally replaced by a fluorine atom and which optionally contains an ether bond in a structure.

The production method of the disclosure preferably further includes:
a purifying step (2) including: adding an acid to a reaction solution containing the compound (2) obtained in the step (1) to achieve a pH of 3 or lower; performing extraction on the reaction solution to provide an extract; and washing the extract with water to remove a metal compound; or
a purifying step (3) including: adding an acid to a reaction solution containing the compound (2) obtained in the step (1) to achieve a pH of 3 or lower; and washing a solution at a lower layer with water to remove a metal compound.

The disclosure also relates to a composition containing:
a compound (2);
a metal compound; and
at least one selected from the group consisting of a compound (3) and a compound (4),
the compound (2) being represented by the following formula (2): 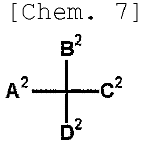 wherein
   A² to D² are each independently
   hydrogen,
   fluorine,
   a hydroxy group,
   a C1-C10 hydrocarbon group in which at least one hydrogen atom is optionally replaced by a fluorine atom and which optionally contains a cyclic structure, a multiple bond, a carbonyl group, or an ether bond in a structure,
   a substituent represented by the following formula (C) : 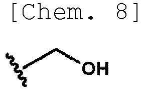 , or
   a substituent represented by the following formula (D) : 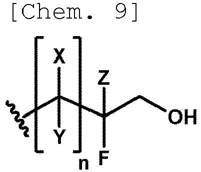
   wherein
      n is 0 to 5; and
      X, Y, and Z are each independently
         hydrogen,
         fluorine, or
         a C1-C10 hydrocarbon group in which at least one hydrogen atom is optionally replaced by a fluorine atom and which optionally contains a cyclic structure, a multiple bond, a carbonyl group, or an ether bond in a structure,
   not all of A² to D² are substituents represented by the formula (C),
   at least one selected from A² to D² is a substituent represented by the formula (C) or the formula (D), and
   in the case where one to three selected from A² to D² are substituents represented by the formula (C), one or more of remaining substituents are fluorine,
   the compound (3) being represented by the following formula (3): 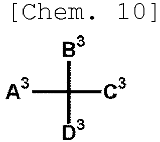 wherein
      A³ to D³ are each independently
         hydrogen,
         fluorine,
         a hydroxy group,
         a C1-C10 hydrocarbon group in which at least one hydrogen atom is optionally replaced by a fluorine atom and which optionally contains a cyclic structure, a multiple bond, a carbonyl group, or an ether bond in a structure,
      a substituent represented by the following formula (E) : 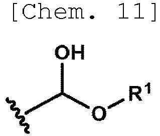
      wherein R¹ is hydrogen or a C1-C10 hydrocarbon group in which at least one hydrogen atom is optionally replaced by a fluorine atom and which optionally contains a cyclic structure, a multiple bond, a carbonyl group, or an ether bond in a structure, or
         a substituent represented by the following formula (F): 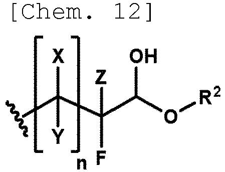
      wherein
         n is 0 to 5;
         X, Y, and Z are each independently
            hydrogen,
            fluorine, or
            a C1-C10 hydrocarbon group in which at least one hydrogen atom is optionally replaced by a fluorine atom and which optionally contains a cyclic structure, a multiple bond, a carbonyl group, or an ether bond in a structure; and
         R² is hydrogen or a C1-C10 hydrocarbon group in which at least one hydrogen atom is optionally replaced by a fluorine atom and which optionally contains a cyclic structure, a multiple bond, a carbonyl group, or an ether bond in a structure,
      not all of A³ to D³ are substituents represented by the formula (E),
      at least one selected from A³ to D³ is a substituent represented by the formula (E) or the formula (F),
      in the case where one to three selected from A³ to D³ are substituents represented by the formula (E), one or more of remaining substituents are fluorine, and
      in the case where two or three selected from A³ to D³ are substituents each represented by the formula (E) or the formula (F), R¹ and R² are optionally bonded to each other at one or more carbon atoms each at any position,
      the compound (4) being represented by the following formula (4): 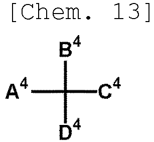 wherein
         A⁴ to D⁴ are each independently
         hydrogen,
         fluorine,
         a hydroxy group,
         a C1-C10 hydrocarbon group in which at least one hydrogen atom is optionally replaced by a fluorine atom and which optionally contains a cyclic structure, a multiple bond, a carbonyl group, or an ether bond in a structure,
         a substituent represented by the following formula (G) : 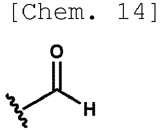 , or
         a substituent represented by the following formula (H) : 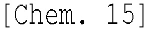 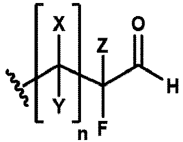
         wherein
            n is 0 to 5; and
            X, Y, and Z are each independently
               hydrogen,
               fluorine, or
               a C1-C10 hydrocarbon group in which at least one hydrogen atom is optionally replaced by a fluorine atom and which optionally contains a cyclic structure, a multiple bond, a carbonyl group, or an ether bond in a structure,
         not all of A⁴ to D⁴ are substituents represented by the formula (G),
         at least one selected from A⁴ to D⁴ is a substituent represented by the formula (G) or the formula (H), and
         in the case where one to three selected from A⁴ to D⁴ are substituents represented by the formula (G), one or more of remaining substituents are fluorine.

In the composition of the disclosure, the metal compound contains a metal element in an amount of preferably 10000 ppm or less, more preferably 3000 ppm or less relative to the compound (2).

In the composition of the disclosure, the compound (3) is contained in an amount of preferably 10000 ppm or less, more preferably 1000 ppm or less, still more preferably 300 ppm or less relative to the compound (2).

In the composition of the disclosure, the compound (4) is contained in an amount of preferably 10000 ppm or less, more preferably 1000 ppm or less, still more preferably 300 ppm or less relative to the compound (2).

The disclosure also relates to a composition containing water and a compound (2), the water being contained in an amount of 1500 ppm or less relative to the compound (2),
the compound (2) being represented by the following formula (2): 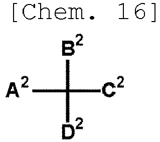 wherein
A² to D² are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (C) : 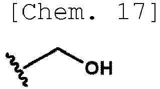 , or
a substituent represented by the following formula (D) : 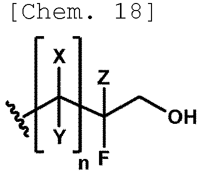
wherein
   n is 0 to 5; and
   X, Y, and Z are each independently
      hydrogen,
      fluorine, or
      a C1-C10 hydrocarbon group in which at least one hydrogen atom is optionally replaced by a fluorine atom and which optionally contains a cyclic structure, a multiple bond, a carbonyl group, or an ether bond in a structure,
not all of A² to D² are substituents represented by the formula (C),
at least one selected from A² to D² is a substituent represented by the formula (C) or the formula (D), and
in the case where one to three selected from A² to D² are substituents represented by the formula (C), one or more of remaining substituents are fluorine.

In the composition of the disclosure, the water is contained in an amount of preferably 500 ppm or less, more preferably 100 ppm or less relative to the compound (2).

### - Advantageous Effects of Invention

When alcohols obtained by the production method of the disclosure are used as pharmaceutical or agricultural chemicals, they need to contain a reduced amount of impurities so as not to cause functional influences. In this respect, the above structure can successfully reduce the amount of solvents used, reduce the environmental load and cost, and produce an alcohol at high concentration and in high yield.

### DESCRIPTION OF EMBODIMENTS

Conventional alcohol production processes by reduction of esters are performed under conditions where materials are diluted so as to reduce runaway reactions, which provide alcohols unfortunately at low concentration. These production processes also provide a mixture containing reaction intermediates such as hemiacetals and aldehydes, which are difficult to separate. The inventors performed studies to find that using a solvent selected from a bulky protic solvent, water, or a solvent mixture thereof as well as using a small amount of a metal hydride compound, which is highly safe and is applicable to industrial uses, enable synthesis of an alcohol at high concentration and in high yield in a short reaction time without diluting the materials. Thereby, they arrived at development of the production method of the disclosure.

The alcohol production method of the disclosure is specifically described hereinbelow.

The alcohol production method of the disclosure includes a step (1) of reacting a compound (1) represented by the formula (1) and a metal hydride compound under conditions where at least one solvent selected from the group consisting of water and a C3 or higher protic solvent is present and the compound (1) is at a concentration of 1.0 mmol/g (amount of substance of compound (1)/total solvent amount) or higher to provide a compound (2) represented by the following formula (2).

The compound (1) is represented by the formula (1): 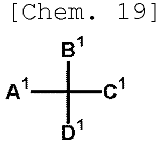 wherein
A¹ to D¹ are each independently
   hydrogen,
   fluorine,
   a hydroxy group,
   a C1-C10 hydrocarbon group in which at least one hydrogen atom is optionally replaced by a fluorine atom and which optionally contains a cyclic structure, a multiple bond, a carbonyl group, or an ether bond in a structure,
   a substituent represented by the following formula (A): 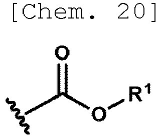
wherein R¹ is hydrogen or a C1-C10 hydrocarbon group in which at least one hydrogen atom is optionally replaced by a fluorine atom and which optionally contains a cyclic structure, a multiple bond, a carbonyl group, or an ether bond in a structure, or
   a substituent represented by the following formula (B): 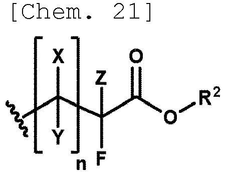
wherein
   n is 0 to 5;
   X, Y, and Z are each independently
      hydrogen,
      fluorine, or
      a C1-C10 hydrocarbon group in which at least one hydrogen atom is optionally replaced by a fluorine atom and which optionally contains a cyclic structure, a multiple bond, a carbonyl group, or an ether bond in a structure; and
   R² is hydrogen or a C1-C10 hydrocarbon group in which at least one hydrogen atom is optionally replaced by a fluorine atom and which optionally contains a cyclic structure, a multiple bond, a carbonyl group, or an ether bond in a structure,
not all of A¹ to D¹ are substituents represented by the formula (A),
at least one selected from A¹ to D¹ is a substituent represented by the formula (A) or the formula (B),
in the case where one to three selected from A¹ to D¹ are substituents represented by the formula (A), one or more of the remaining substituents are fluorine, and
in the case where two or three selected from A¹ to D¹ are substituents each represented by the formula (A) or the formula (B), R¹ and R² are optionally bonded to each other at one or more carbon atoms each at any position.

Examples of the cyclic structure include aromatic rings such as a benzene ring and nonaromatic rings such as cyclohexane. Examples of the multiple bond include a double bond and a triple bond. Examples of the C1-C10 hydrocarbon group in which at least one hydrogen atom is optionally replaced by a fluorine atom and which optionally contains a cyclic structure, a multiple bond, a carbonyl group, or an ether bond in the structure include a methyl group, an ethyl group, a n-propyl group, an isopropyl group, a n-butyl group, a sec-butyl group, an isobutyl group, a tert-butyl group, a n-pentyl group, a sec-pentyl group, an isopentyl group, a neopentyl group, a hexyl group, a 2-methylpentyl group, and a heptyl group in which at least one hydrogen atom is optionally replaced by a fluorine atom and which optionally contains a carbonyl group or an ether bond in the structure.

In the formula, n is 0 to 5. In order to provide an alcohol at higher concentration and in higher yield, n is preferably 0 to 3.

In order to provide an alcohol at higher concentration and in higher yield, preferably, X, Y, and Z are each independently hydrogen, fluorine, or a C1-C5 hydrocarbon group in which at least one hydrogen atom is optionally replaced by a fluorine atom, more preferably hydrogen or fluorine.

In order to provide an alcohol at higher concentration and in higher yield, preferably, n is 0 to 3 and X, Y, and Z are each independently hydrogen or fluorine.

In order to provide an alcohol at higher concentration and in higher yield, R¹ or R² is preferably a methyl group, an ethyl group, a n-propyl group, an isopropyl group, a n-butyl group, a sec-butyl group, an isobutyl group, a tert-butyl group, a n-pentyl group, a sec-pentyl group, an isopentyl group, a neopentyl group, a hexyl group, a 2-methylpentyl group, or a heptyl group in which at least one hydrogen atom is optionally replaced by a fluorine atom and which optionally contains an ether bond in the structure, more preferably a methyl group, an ethyl group, a n-propyl group, an isopropyl group, a n-butyl group, a sec-butyl group, an isobutyl group, a tert-butyl group, a 2-methylpentyl group, or a heptyl group in which at least one hydrogen atom is optionally replaced by a fluorine atom and which optionally contains an ether bond in the structure, still more preferably a methyl group, an ethyl group, a n-propyl group, an isopropyl group, a tert-butyl group, or a 2-methylpentyl group in which at least one hydrogen atom is optionally replaced by a fluorine atom and which optionally contains an ether bond in the structure.

In order to provide an alcohol at higher concentration and in higher yield, the substituent represented by the formula (A) is preferably a methoxycarbonyl group, an ethoxycarbonyl group, a n-propoxycarbonyl group, an isopropoxycarbonyl group, a n-butoxycarbonyl group, a sec-butoxycarbonyl group, an isobutoxycarbonyl group, a tert-butoxycarbonyl group, a n-pentoxycarbonyl group, a sec-pentoxycarbonyl group, an isopentoxycarbonyl group, a neopentoxycarbonyl group, a hexoxycarbonyl group, a heptoxycarbonyl group, a 2-methoxyethoxycarbonyl group, a 2-(2-methoxyethoxy)ethoxycarbonyl group, a 2-propoxypropoxycarbonyl group, a trifluoromethoxycarbonyl group, a 2,2,2-trifluoroethoxycarbonyl group, a perfluoroethoxycarbonyl group, a perfluoro-n-propoxycarbonyl group, a 2,2,3,3,3-pentafluoro-n-propoxycarbonyl group, a perfluoroisopropoxycarbonyl group, a 1,1,1,3,3,3-hexafluoroisopropoxycarbonyl group, a perfluoro-2-ethoxypropoxycarbonyl group, a perfluoro-2-propoxypropoxycarbonyl group, a perfluoro-2-butoxypropoxycarbonyl group, a perfluoro-2-ethoxybutoxycarbonyl group, a perfluoro-2-propoxybutoxycarbonyl group, or a perfluoro-2-butoxybutoxycarbonyl group, more preferably a methoxycarbonyl group, an ethoxycarbonyl group, a n-propoxycarbonyl group, an isopropoxycarbonyl group, a tert-butoxycarbonyl group, a 2-methoxyethoxycarbonyl group, a 2-propoxypropoxycarbonyl group, a 2,2,2-trifluoroethoxycarbonyl group, a 2,2,3,3,3-pentafluoro-n-propoxycarbonyl group, a perfluoro-2-ethoxypropoxycarbonyl group, or a perfluoro-2-propoxypropoxycarbonyl group.

In order to provide an alcohol at higher concentration and in higher yield, the substituent represented by the formula (B) is preferably any of the following.

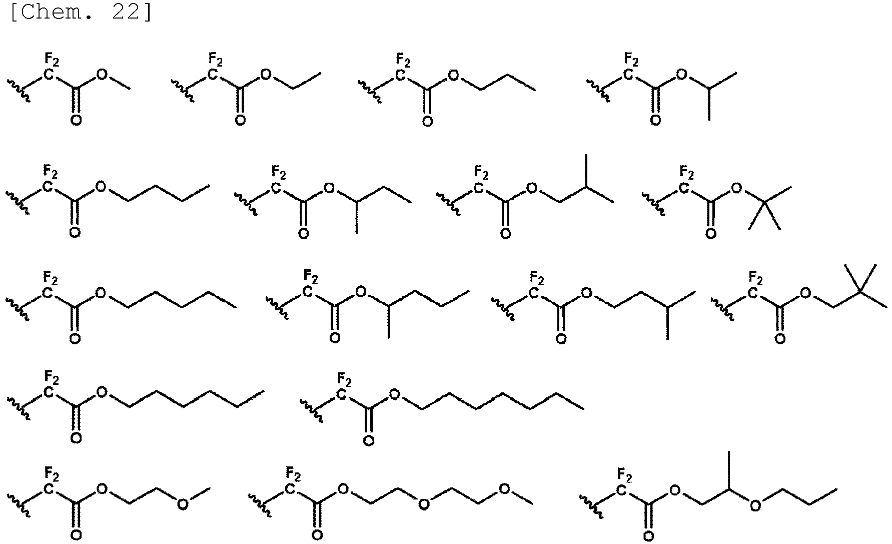

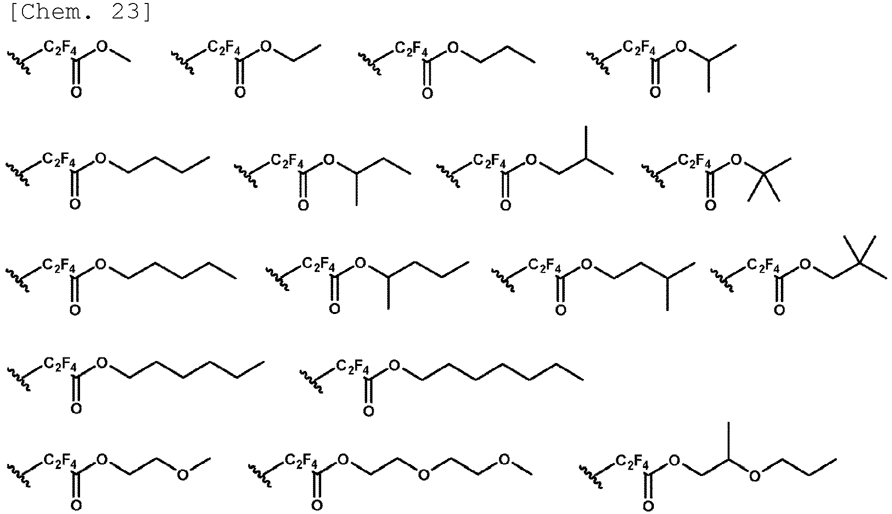

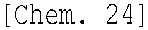

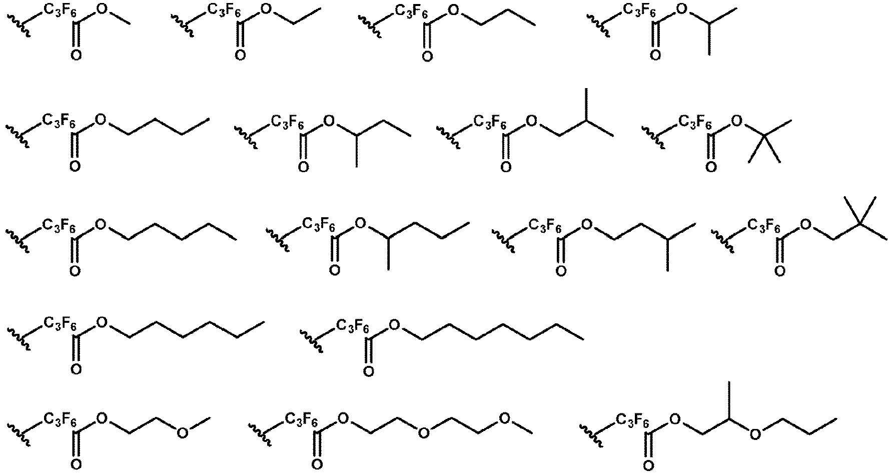

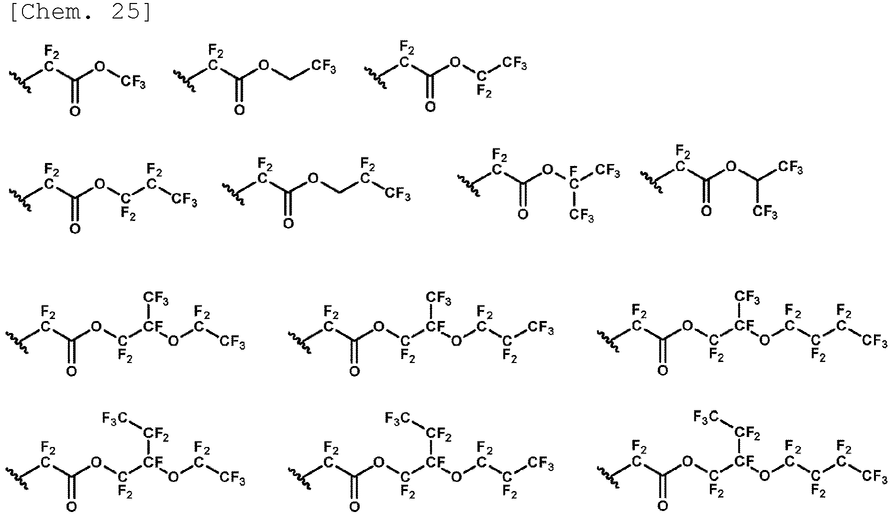

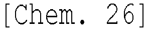

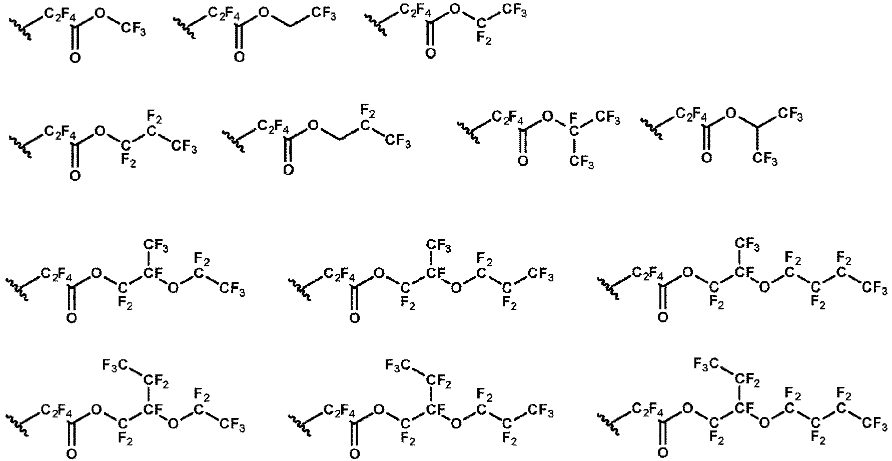

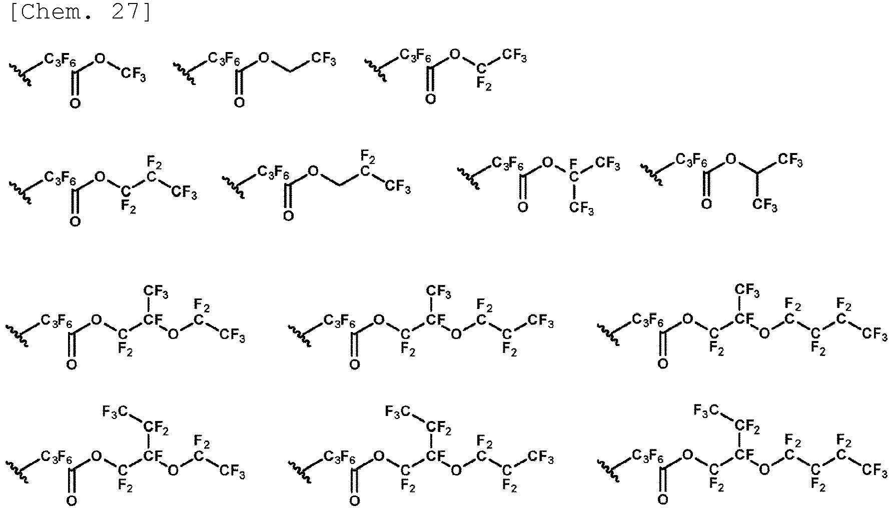

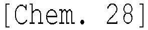

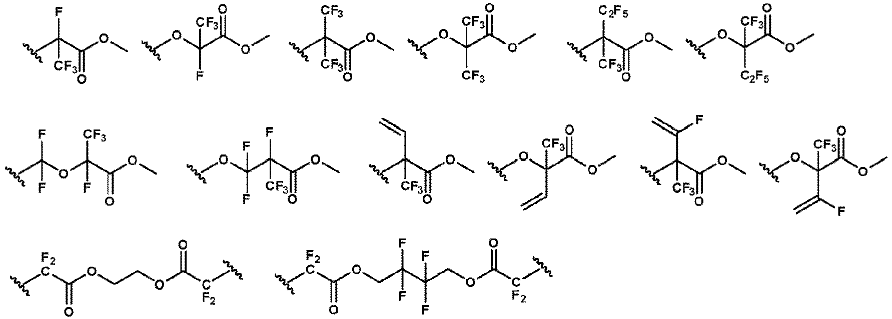

Not all of A¹ to D¹ are substituents represented by the formula (A). At least one selected from A¹ to D¹ is a substituent represented by the formula (A) or the formula (B). In the case where one to three selected from A¹ to D¹ are substituents represented by the formula (A), one or more of the remaining substituents are fluorine. In the case where two or three selected from A¹ to D¹ are substituents each represented by the formula (A) or the formula (B), R¹ and R² are optionally bonded to each other at one or more carbon atoms each at any position.

Preferably, one or two selected from A¹ to D¹ are substituents represented by the formula (A) and another one or two selected from A¹ to D¹ are fluorine. Preferred combinations of A¹ to D¹ include a combination in which one selected from A¹ to D¹ is a substituent represented by the formula (A), another selected from A¹ to D¹ is a substituent represented by the formula (B), and the other two remaining substituents are fluorine; a combination in which two selected from A¹ to D¹ are substituents represented by the formula (A), another selected from A¹ to D¹ is fluorine, and the other remaining substituent is hydrogen; and a combination in which one selected from A¹ to D¹ is a substituent represented by the formula (A), another selected from A¹ to D¹ is fluorine, and the other two remaining substituents are each a C1-C10 hydrocarbon group in which at least one hydrogen atom is optionally replaced by a fluorine atom and which optionally contains a cyclic structure, a multiple bond, a carbonyl group, or an ether bond in the structure.

Specific examples of the compound (1) include methyl 2,3,3,3-tetrafluoro-2-(trifluoromethyl)propanoate, 2,2,2-trifluoroethyl trifluoroacetate, 2,2,2-trifluoroethyl 2-fluoro-3-oxobutanate, methyl 3,3,3-trifluoro-2-hydroxy-2-trifluoromethyl propanoate, methyl 2,3,3,3-tetrafluoro-2-(1,1,2-trifluoroallyloxy)propanoate, methyl 2,3,3,3-tetrafluoro-2-(1,1,2,2-tetrafluoro-2-(1,2,2-trifluorovinyloxy)ethoxy)propanoate, dimethyl fluoromalonate, diethyl fluoromalonate, dimethyl tetrafluorosuccinate, diethyl tetrafluorosuccinate, dimethyl hexafluoroglutarate, diethyl hexafluoroglutarate, dimethyl octafluoroadipate, and diethyl octafluoroadipate.

In order to provide an alcohol at higher concentration and in higher yield, the metal hydride compound preferably includes at least one selected from the group consisting of a boron hydride compound and an aluminum hydride compound, more preferably includes at least one selected from the group consisting of lithium borohydride, lithium aminoborohydride, lithium triethylborohydride, lithium tri(sec-butyl)borohydride, potassium borohydride, potassium tri(sec-butyl)borohydride, calcium borohydride, sodium borohydride, sodium cyanoborohydride, sodium triacetoxyborohydride, sodium bis(2-methoxyethoxy)aluminum hydride, zinc borohydride, nickel borohydride, and diborane, still more preferably includes at least one selected from the group consisting of sodium borohydride, sodium cyanoborohydride, and sodium triacetoxyborohydride.

In order to provide an alcohol at higher concentration and in higher yield, the metal hydride compound is used in an amount of preferably less than 2.5 equivalents, more preferably 1.5 equivalents or less, still more preferably 1.2 equivalents or less relative to the ester group in the compound (1).

The step (1) is performed under conditions where at least one solvent selected from the group consisting of water and a C3 or higher protic solvent is present and the compound (1) is at a concentration of 1.0 mmol/g (amount of substance of compound (1)/total solvent amount) or higher.

The protic solvent has a carbon number of 3 or greater. The protic solvent having a carbon number within this range can reduce runaway reactions in production of an alcohol from materials at high concentration. The carbon number is preferably 15 or smaller, more preferably 10 or smaller, still more preferably 5 or smaller. In order to provide an alcohol at higher concentration and in higher yield, the protic solvent is preferably a secondary or tertiary alcohol.

The protic solvent preferably includes at least one selected from the group consisting of n-propyl alcohol, isopropyl alcohol, n-butyl alcohol, sec-butyl alcohol, isobutyl alcohol, tert-butyl alcohol, n-pentyl alcohol, sec-pentyl alcohol, isopentyl alcohol, and neopentyl alcohol. In order to provide an alcohol at higher concentration and in higher yield, more preferred is at least one selected from the group consisting of isopropyl alcohol and tert-butyl alcohol.

In the step (1), a different solvent may be contained in addition to the at least one selected from water and a C3 or higher protic solvent. Examples of the different solvent include linear monoethers such as diethyl ether, methyl-tert-butyl ether, diisopropyl ether, cyclopentyl methyl ether, heptafluoromethyl ether, nonafluorobutyl methyl ether, and nonafluorobutyl ethyl ether, glymes such as monoglyme, diglyme, triglyme, and tetraglyme, cyclic ethers such as tetrahydrofuran and 1,4-dioxane, and fluorinated oils.

The compound (1) is at a concentration of 1.0 mmol/g or higher relative to the total solvent amount. The total solvent amount means the sum of the masses of water, C3 or higher protic solvents, and different solvents. The compound (1) at a concentration within this range can successfully reduce the amount of solvents used, reduce the environmental load and cost, and produce an alcohol at high concentration and in high yield. In order to provide an alcohol at higher concentration and in higher yield, the concentration of the compound (1) is preferably 1.2 mmol/g or higher, more preferably 1.4 mmol/g or higher relative to the total solvent amount. In order to reduce an increase in the viscosity of the reaction solution, the concentration is preferably 10 mmol/g or lower, more preferably 5 mmol/g or lower.

In the step (1), preferably 80% by mass or more of the total solvent amount corresponds to at least one solvent selected from the group consisting of water and a C3 or higher protic solvent, more preferably 90% by mass or more corresponds to at least one solvent selected from the group consisting of water and a C3 or higher protic solvent, still more preferably 95% by mass or more corresponds to at least one solvent selected from the group consisting of water and a C3 or higher protic solvent. Also, preferably 80% by mass or more of the total solvent amount corresponds to a C3 or higher protic solvent, more preferably 90% by mass or more corresponds to a C3 or higher protic solvent, still more preferably 95% by mass or more corresponds to a C3 or higher protic solvent.

The compound (2) is represented by the following formula (2): 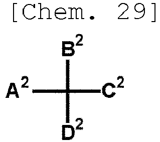 wherein
A² to D² are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (C) : 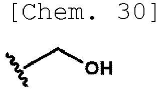 , or
a substituent represented by the following formula (D) : 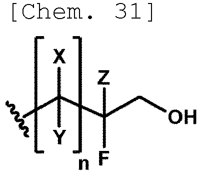
wherein
   n is 0 to 5; and
   X, Y, and Z are each independently
      hydrogen,
      fluorine, or
      a C1-C10 hydrocarbon group in which at least one hydrogen atom is optionally replaced by a fluorine atom and which optionally contains a cyclic structure, a multiple bond, a carbonyl group, or an ether bond in a structure,
not all of A² to D² are substituents represented by the formula (C),
at least one selected from A² to D² is a substituent represented by the formula (C) or the formula (D), and
in the case where one to three selected from A² to D² are substituents represented by the formula (C), one or more of the remaining substituents are fluorine.

In the formulas, n, X, Y, and Z are as described for the formula (B).

The substituent represented by the formula (D) is preferably any of the following.

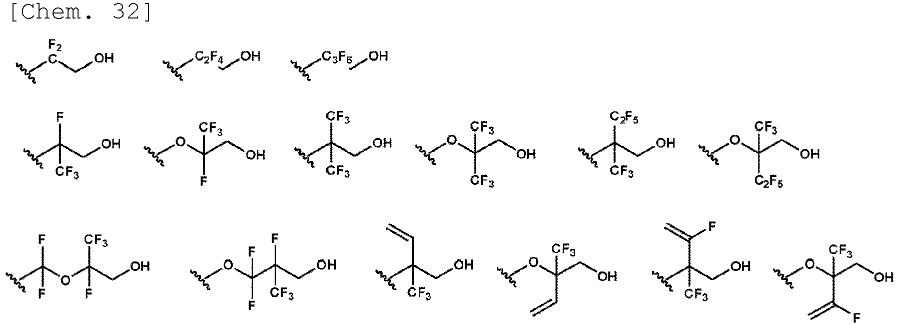

Not all of A² to D² are substituents represented by the formula (C). At least one selected from A² to D² is a substituent represented by the formula (C) or the formula (D). In the case where one to three selected from A² to D² are substituents represented by the formula (C), one or more of the remaining substituents are fluorine.

Preferably, one or two selected from A² to D² are substituents represented by the formula (C) and another one or two selected from A² to D² are fluorine. Preferred combinations of A² to D² include a combination in which one selected from A² to D² is a substituent represented by the formula (C), another selected from A² to D² is a substituent represented by the formula (D), and the other two remaining substituents are fluorine; a combination in which two selected from A² to D² are substituents represented by the formula (C), another selected from A² to D² is fluorine, and the other remaining substituent is hydrogen; and a combination in which one selected from A² to D² is a substituent represented by the formula (A), another selected from A² to D² is fluorine, and the other two remaining substituents are each a C1-C10 hydrocarbon group in which at least one hydrogen atom is optionally replaced by a fluorine atom and which optionally contains a cyclic structure, a multiple bond, a carbonyl group, or an ether bond in the structure.

Examples of the compound (2) include 2,3,3,3-tetrafluoro-2-(trifluoroethyl)propan-1-ol, 2,2,2-trifluoroethanol, 3-fluoro-4-hydroxybutan-2-one, 2,3,3,3-tetrafluoro-2-(1,1,2-trifluoroallyloxy)propan-1-ol, 2,3,3,3-tetrafluoro-2-(1,1,2,2-tetrafluoro-2-(1,2,2-trifluorovinyloxy)ethoxy)propan-1-ol, 3,3,3-trifluoro-2-(trifluoromethyl)propane-1,2-diol, 2-fluoropropane-1,3-diol, 2,2,3,3-tetrafluorobutane-1,4-diol, 2,2,3,3,4,4-hexafluoropentane-1,5-diol, and 2,2,3,3,4,4,5,5-octafluorohexane-1,6-diol.

The temperature in the step (1) may be, but is not limited to, 100°C or lower, preferably 85°C to 0°C.

The duration of the step (1) is commonly, but not limited to, 0.1 to 120 minutes, preferably 15 to 90 minutes.

The production method of the disclosure preferably further includes:
a purifying step (2) including: adding an acid to a reaction solution containing the compound (2) obtained in the step (1) to achieve a pH of 3 or lower; performing extraction on the reaction solution to provide an extract; and washing the extract with water to remove a metal compound; or
a purifying step (3) including: adding an acid to a reaction solution containing the compound (2) obtained in the step (1) to achieve a pH of 3 or lower; and washing a solution at a lower layer with water to remove a metal compound.

Examples of the acid include hydrochloric acid, hydrofluoric acid, bromic acid, sulfuric acid, and nitric acid.

The metal compound is one generated by oxidation of the metal hydride compound in the step (1), and preferably includes at least one selected from the group consisting of a boron compound and an aluminum compound, such as boric acid and aluminum hydroxide.

The temperature in the step (2) or (3) may be, but is not limited to, 50°C or lower, preferably 30°C to 0°C.

The duration of the step (2) or (3) is commonly, but not limited to, 0.1 to 120 minutes, preferably 10 to 60 minutes.

The production method of the disclosure provides the compound (2) in a yield of preferably 30% or higher, more preferably 50% or higher, still more preferably 70% or higher. The yield is calculated by (amount of substance of compound (2))/(amount of substance of compound (1)).

The disclosure also provides a composition obtainable by the above production method. The disclosure also provides a composition (hereinafter, also referred to as a "first composition of the disclosure") containing the compound (2), the metal compound, and at least one selected from the group consisting of a compound (3) represented by the formula (3) and a compound (4) represented by the formula (4). The first composition of the disclosure is obtainable by the above production method.

In the first composition of the disclosure, the compound (2) and the metal compound are as described for the above production method.

In the first composition of the disclosure, the compound (3) is represented by the following formula (3): 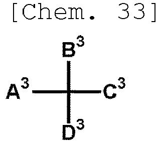 wherein
A³ to D³ are each independently
   hydrogen,
   fluorine,
   a hydroxy group,
   a C1-C10 hydrocarbon group in which at least one hydrogen atom is optionally replaced by a fluorine atom and which optionally contains a cyclic structure, a multiple bond, a carbonyl group, or an ether bond in a structure,
   a substituent represented by the following formula (E): 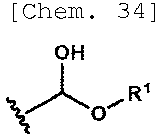
wherein R¹ is hydrogen or a C1-C10 hydrocarbon group in which at least one hydrogen atom is optionally replaced by a fluorine atom and which optionally contains a cyclic structure, a multiple bond, a carbonyl group, or an ether bond in a structure, or
   a substituent represented by the following formula (F): 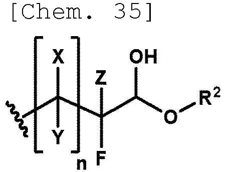
wherein
   n is 0 to 5;
   X, Y, and Z are each independently
      hydrogen,
      fluorine, or
      a C1-C10 hydrocarbon group in which at least one hydrogen atom is optionally replaced by a fluorine atom and which optionally contains a cyclic structure, a multiple bond, a carbonyl group, or an ether bond in a structure; and
   R² is hydrogen or a C1-C10 hydrocarbon group in which at least one hydrogen atom is optionally replaced by a fluorine atom and which optionally contains a cyclic structure, a multiple bond, a carbonyl group, or an ether bond in a structure,
not all of A³ to D³ are substituents represented by the formula (E),
at least one selected from A³ to D³ is a substituent represented by the formula (E) or the formula (F),
in the case where one to three selected from A³ to D³ are substituents represented by the formula (E), one or more of the remaining substituents are fluorine, and
in the case where two or three selected from A³ to D³ are substituents each represented by the formula (E) or the formula (F), R¹ and R² are optionally bonded to each other at one or more carbon atoms each at any position.

R¹, R², n, X, Y, and Z are as described for the formulas (A) and (B).

The substituent represented by the formula (E) is preferably any of the following.

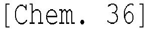

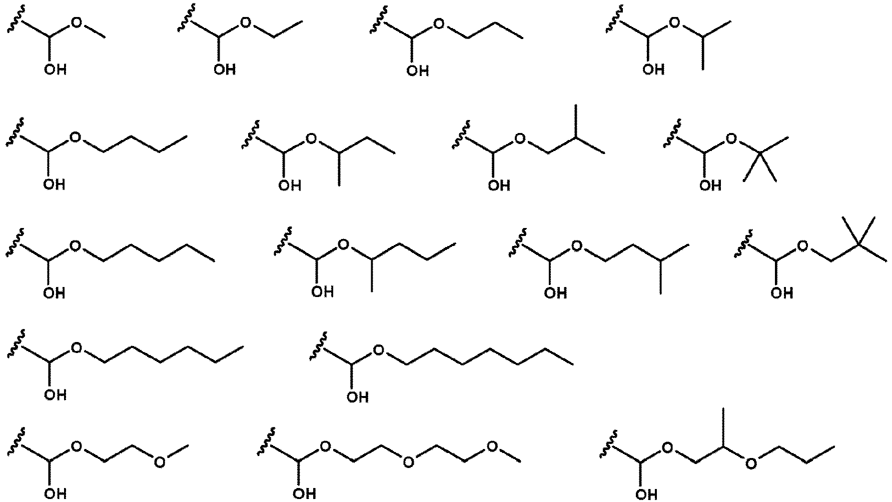

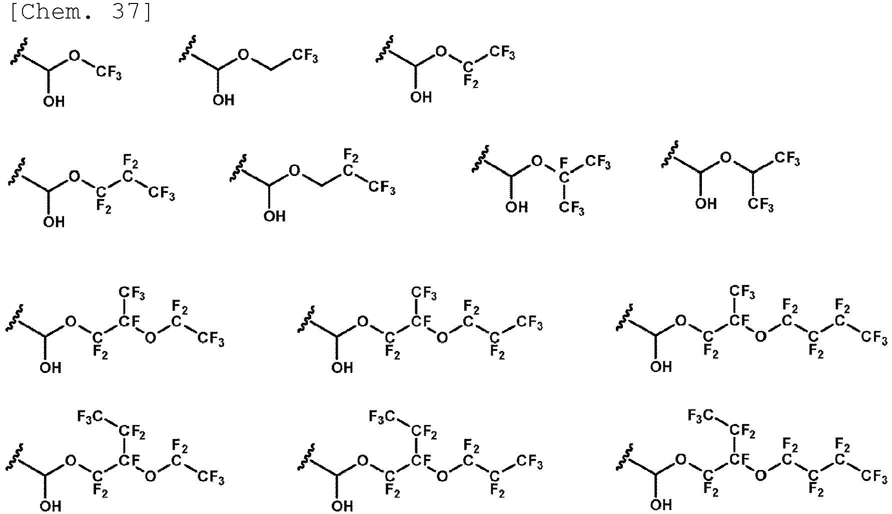

More preferred is any of the following.

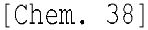

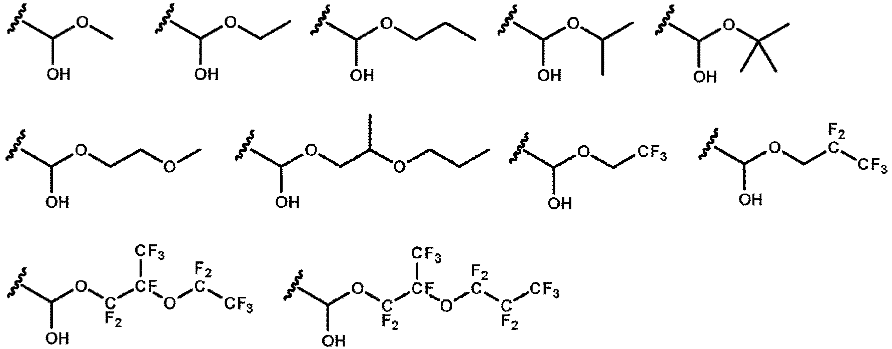

The substituent represented by the formula (F) is preferably any of the following.

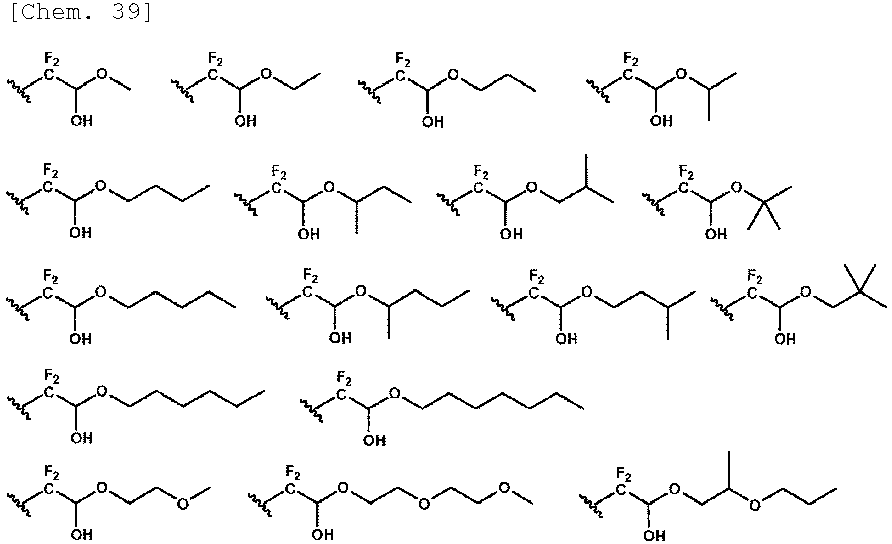

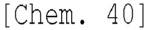

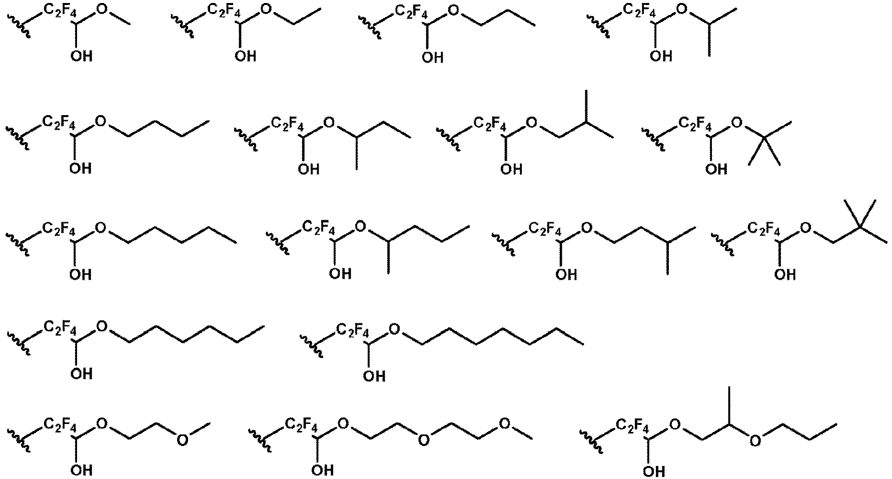

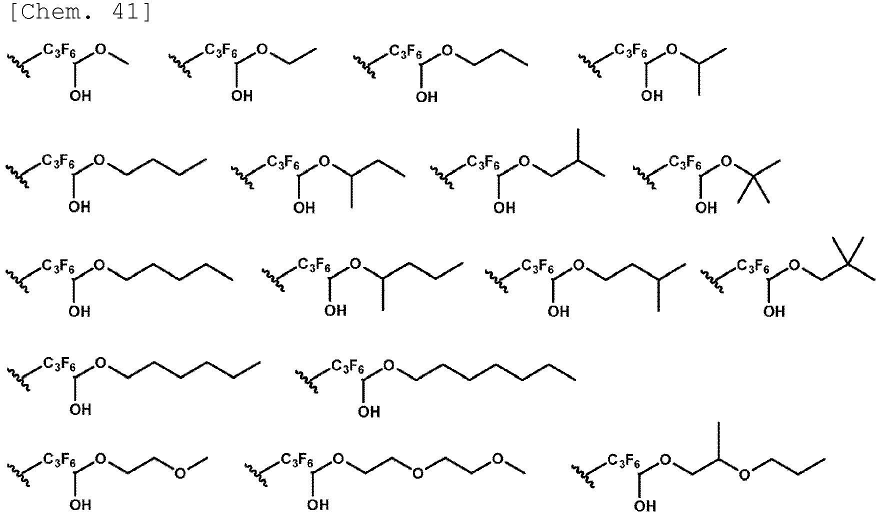

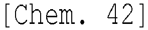

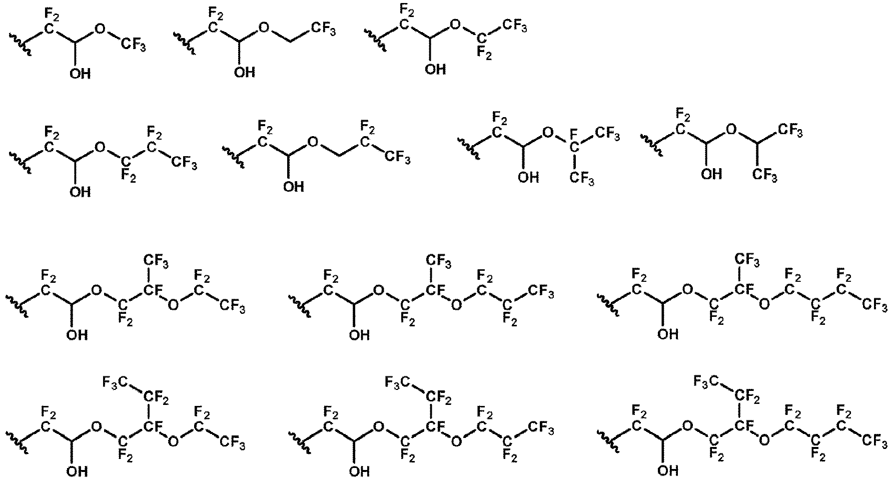

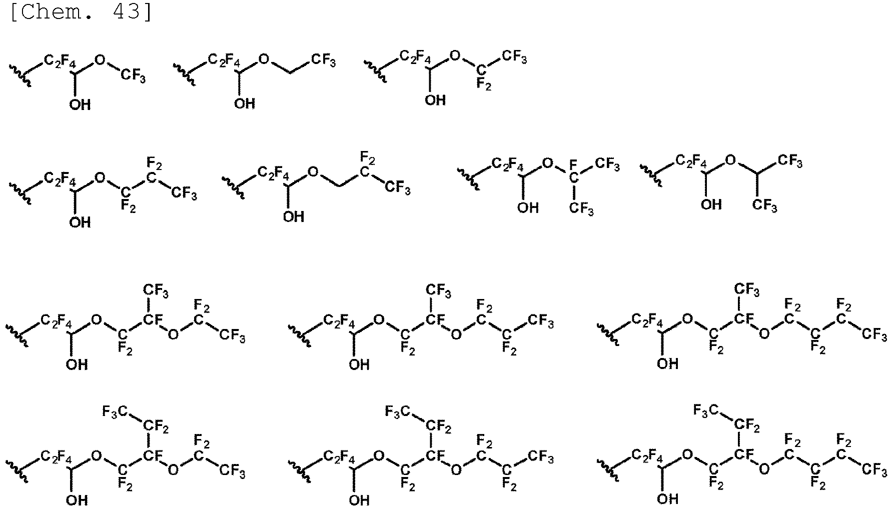

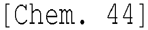

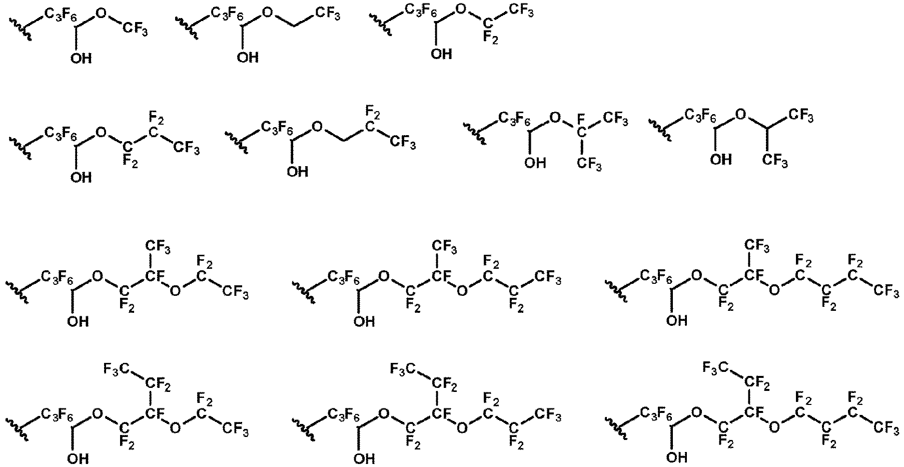

More preferred is any of the following.

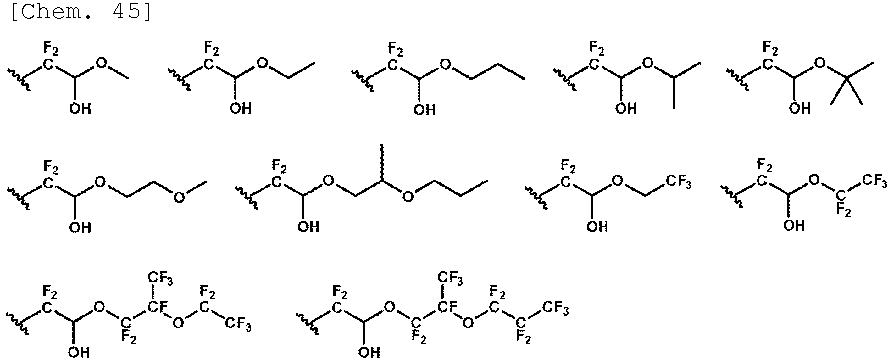

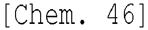

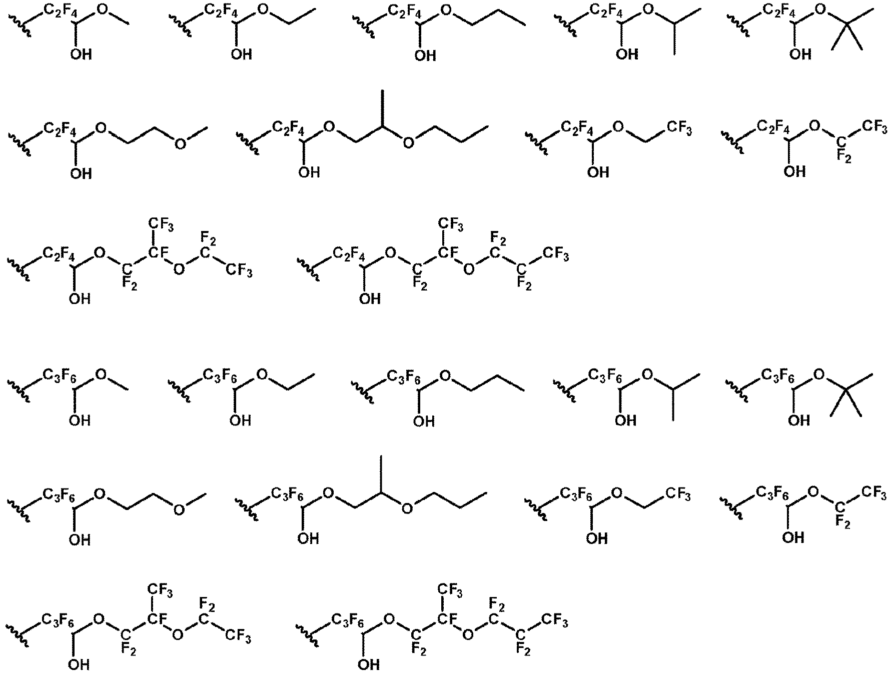

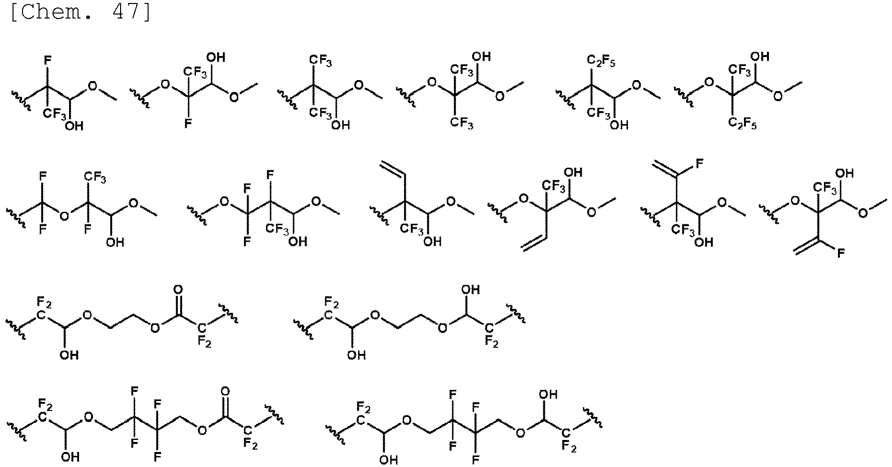

Not all of A³ to D³ are substituents represented by the formula (E). At least one selected from A³ to D³ is a substituent represented by the formula (E) or the formula (F). In the case where one to three selected from A³ to D³ are substituents represented by the formula (E), one or more of the remaining substituents are fluorine. In the case where two or three selected from A³ to D³ are substituents each represented by the formula (E) or the formula (F), R¹ and R² are optionally bonded to each other at one or more carbon atoms each at any position.

Specific examples of the compound (3) include the following.

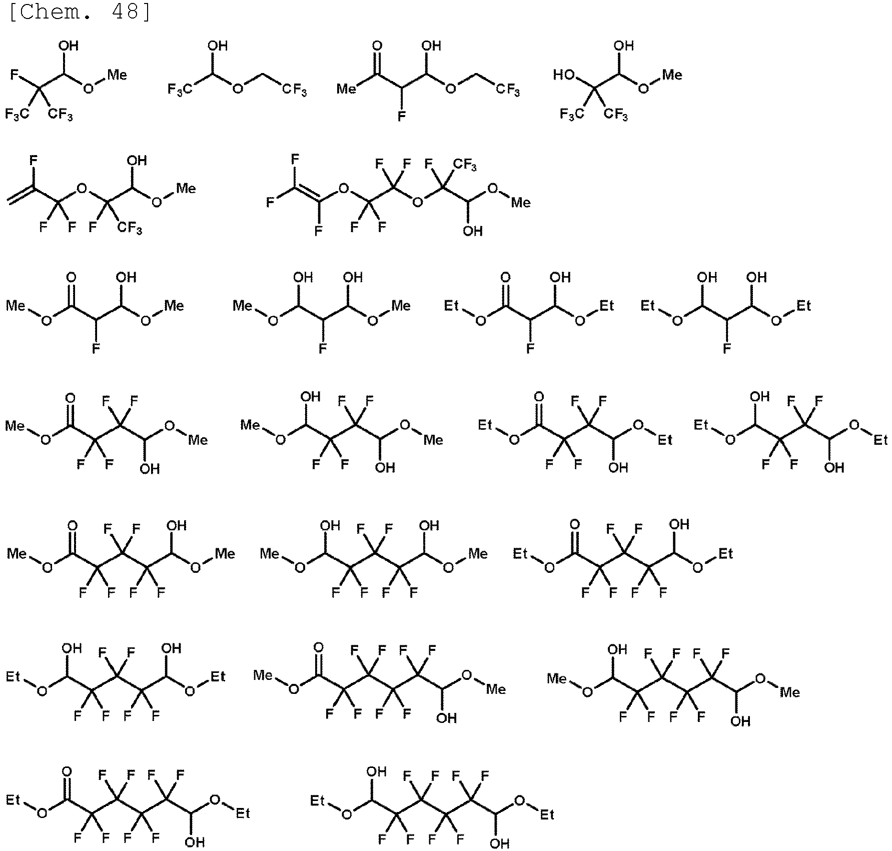

In the first composition of the disclosure, the compound (4) is represented by the following formula (4): 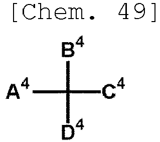 wherein
A⁴ to D⁴ are each independently
hydrogen,
fluorine,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (G) : 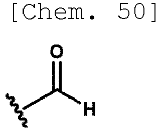 , or
a substituent represented by the following formula (H) : 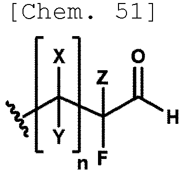
wherein
   n is 0 to 5; and
   X, Y, and Z are each independently
      hydrogen,
      fluorine, or
      a C1-C10 hydrocarbon group in which at least one hydrogen atom is optionally replaced by a fluorine atom and which optionally contains a cyclic structure, a multiple bond, a carbonyl group, or an ether bond in a structure,
not all of A⁴ to D⁴ are substituents represented by the formula (G),
at least one selected from A⁴ to D⁴ is a substituent represented by the formula (G) or the formula (H), and
in the case where one to three selected from A⁴ to D⁴ are substituents represented by the formula (G), one or more of the remaining substituents are fluorine.

In the formula, n, X, Y, and Z are as described for the formula (B).

The substituent represented by the formula (H) is preferably any of the following.

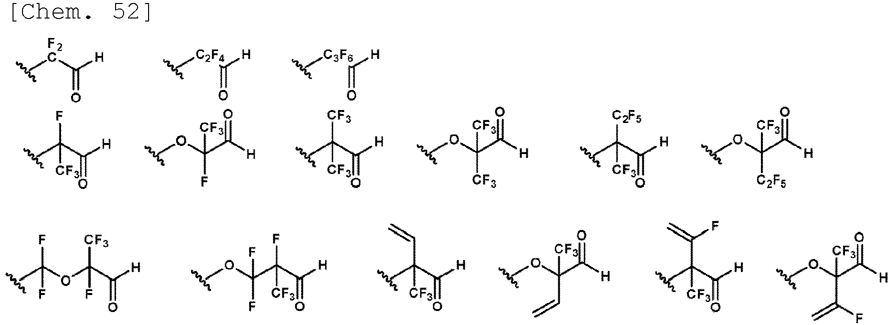

Not all of A⁴ to D⁴ are substituents represented by the formula (G). At least one selected from A⁴ to D⁴ is a substituent represented by the formula (G) or the formula (H). In the case where one to three selected from A⁴ to D⁴ are substituents represented by the formula (G), one or more of the remaining substituents are fluorine.

Specific examples of the compound (4) include the following.

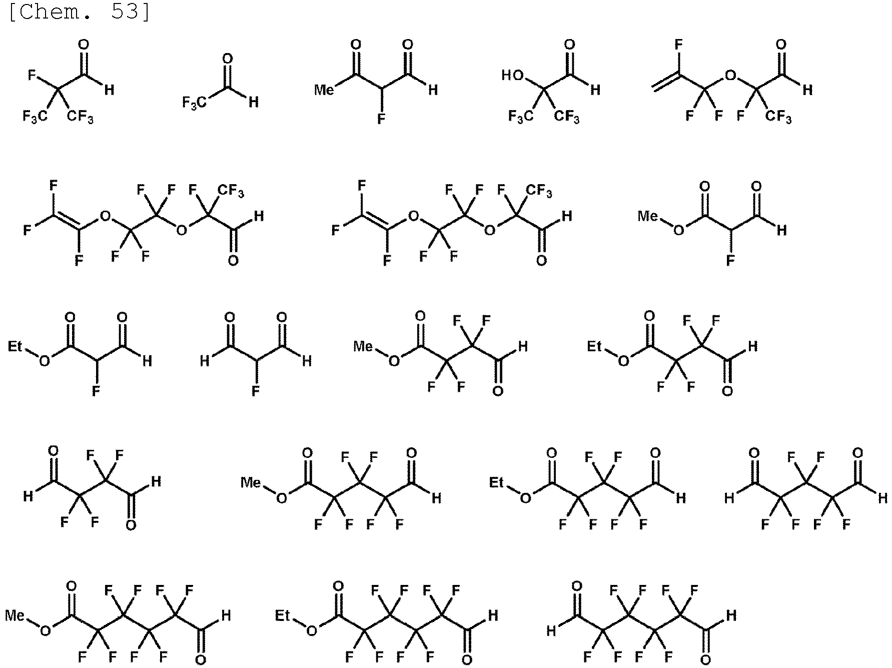

In the first composition of the disclosure, the metal element contained in the metal compound is contained in an amount of preferably 10000 ppm or less, more preferably 3000 ppm or less, still more preferably 2000 ppm or less relative to the compound (2). The lower limit of the amount of the metal element contained in the metal compound may be, but is not limited to, 0.001 ppm. The amount of the metal element contained in the metal compound can be determined by elemental analysis.

In the first composition of the disclosure, the compound (3) is contained in an amount of preferably 10000 ppm or less, more preferably 1000 ppm or less, still more preferably 300 ppm or less relative to the compound (2). The lower limit of the amount of the compound (3) contained may be, but is not limited to, 0.001 ppm. The amount of the compound (3) contained can be determined by ¹⁹F-NMR, ¹H-NMR, GC, or LC.

In the first composition of the disclosure, the compound (4) is contained in an amount of preferably 10000 ppm or less, more preferably 1000 ppm or less, still more preferably 300 ppm or less relative to the compound (2). The lower limit of the amount of the compound (4) contained may be, but is not limited to, 0.001 ppm. The amount of the compound (4) contained can be determined by ¹⁹F-NMR, ¹H-NMR, GC, or LC.

The first composition of the disclosure may further contain water. In the first composition of the disclosure, the water is contained in an amount of preferably 1500 ppm or less, more preferably 500 ppm or less, still more preferably 100 ppm or less relative to the compound (2). The lower limit of the water contained may be, but is not limited to, 0.1 ppm. The amount of the water contained can be determined by the Karl Fischer method.

The disclosure also provides a composition (hereinafter, also referred to as a "second composition of the disclosure") containing the compound (2) and water, the water being contained in an amount of 1500 ppm or less relative to the compound (2). The second composition of the disclosure is obtainable by the above production method.

In the second composition of the disclosure, the compound (2) is as described for the above production method.

In the second composition of the disclosure, the water is contained in an amount of preferably 500 ppm or less, more preferably 100 ppm or less relative to the compound (2). The lower limit of the water contained may be, but is not limited to, 0.1 ppm. The amount of the water contained can be determined by the Karl Fischer method.

The second composition of the disclosure may further contain the metal compound, the compound (3), and the compound (4) in addition to the compound (2) and water. In the second composition of the disclosure, the metal compound is as described for the above production method. The compound (3) and the compound (4) are as described for the first composition of the disclosure.

In the second composition of the disclosure, the metal element contained in the metal compound is contained in an amount of preferably 10000 ppm or less, more preferably 3000 ppm or less, still more preferably 2000 ppm or less relative to the compound (2). The lower limit of the amount of the metal element contained in the metal compound may be, but is not limited to, 0.001 ppm. The amount of the metal element contained in the metal compound can be determined by elemental analysis.

In the second composition of the disclosure, the compound (3) is contained in an amount of preferably 10000 ppm or less, more preferably 3000 ppm or less, still more preferably 1000 ppm or less, further preferably 300 ppm or less relative to the compound (2). The lower limit of the amount of the compound (3) contained may be, but is not limited to, 0.001 ppm. The amount of the compound (3) contained can be determined by ¹⁹F-NMR, ¹H-NMR, GC, or LC.

In the second composition of the disclosure, the compound (4) is contained in an amount of preferably 10000 ppm or less, more preferably 3000 ppm or less, still more preferably 1000 ppm or less, further preferably 300 ppm or less relative to the compound (2). The lower limit of the amount of the compound (4) contained may be, but is not limited to, 0.001 ppm. The amount of the compound (4) contained can be determined by ¹⁹F-NMR, ¹H-NMR, GC, or LC.

The first and second compositions of the disclosure are suitably usable as fluorine-containing materials such as a lens, a prism, a display material, an optical fiber, an optical waveguide material, a coating material, an anti-reflective coating material, a photoresponsive coating material, an adhesive, a 3D fabrication material, a resist material, a sealing agent for circuit boards, a covering material, a sealant, a packaging material, a binder for capacitors and batteries, an additive for capacitors and batteries, an ink, an aerosol agent, a dispersant, and an acid generator, or raw materials thereof.

### EXAMPLES

The disclosure is described in more detail below with reference to examples, but is not limited to these examples.

### Comparative Example 1: production of 2-fluoropropane-1,3-diol

A reactor was charged with sodium borohydride (0.42 g) and methanol (MeOH) (1.0 g), followed by dropwise addition of a compound (1-1): diethyl fluoromalonate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, but a target compound (2-1): 2-fluoropropane-1,3-diol was not obtained. The results are shown in Table 1.

### Example 1: production of 2,2,3,3-tetrafluorobutane-1,4-diol

A reactor was charged with sodium borohydride (0.35 g) and tert-butyl alcohol (t-BuOH) (1.0 g), followed by dropwise addition of a compound (1-2): dimethyl tetrafluorosuccinate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-2): 2,2,3,3-tetrafluorobutane-1,4-diol (0.65 g, yield 87%), 2,2,3,3-tetrafluoro-1,4-dimethoxybutane-1,4-diol + methyl 2,2,3,3-tetrafluoro-4-hydroxy-4-methoxybutanoate (142 ppm in total relative to the compound (2-2)), methyl 2,2,3,3-tetrafluoro-4-oxobutanate (40 ppm relative to the compound (2-2)), and water (433 ppm relative to the compound (2-2)). The results are shown in Table 1.

### Example 2: production of 2,2,3,3-tetrafluorobutane-1,4-diol

An experiment was performed as in Example 1 except that the heat of reaction, generated as in Example 1, was cooled with an ice water bath and the internal temperature was controlled to 50°C or lower. Thereby, a composition was obtained containing the compound (2-2): 2,2,3,3-tetrafluorobutane-1,4-diol (0.64 g, yield 86%), 2,2,3,3-tetrafluoro-1,4-dimethoxybutane-1,4-diol + methyl 2,2,3,3-tetrafluoro-4-hydroxy-4-methoxybutanoate (125 ppm in total relative to the compound (2-2)), methyl 2,2,3,3-tetrafluoro-4-oxobutanate (102 ppm relative to the compound (2-2)), a boron compound (1600 ppm of boron in the boron compound relative to the compound (2-2)), and water (211 ppm relative to the compound (2-2)). The results are shown in Table 1.

### Example 3: production of 2,2,3,3-tetrafluorobutane-1,4-diol

An experiment was performed as in Example 2 except that the amount of t-BuOH used was changed from that in Example 2 to 1.5 g. Thereby, a composition was obtained containing the compound (2-2): 2,2,3,3-tetrafluorobutane-1,4-diol (0.63 g, yield 85%), 2,2,3,3-tetrafluoro-1,4-dimethoxybutane-1,4-diol + methyl 2,2,3,3-tetrafluoro-4-hydroxy-4-methoxybutanoate (98 ppm in total relative to the compound (2-2)), methyl 2,2,3,3-tetrafluoro-4-oxobutanate (91 ppm relative to the compound (2-2)), and water (226 ppm relative to the compound (2-2)). The results are shown in Table 1.

### Example 4: production of 2,2,3,3-tetrafluorobutane-1,4-diol

An experiment was performed as in Example 2 except that the amount of t-BuOH used was changed from that in Example 2 to 3.0 g. Thereby, a composition was obtained containing the compound (2-2): 2,2,3,3-tetrafluorobutane-1,4-diol (0.64 g, yield 86%), 2,2,3,3-tetrafluoro-1,4-dimethoxybutane-1,4-diol + methyl 2,2,3,3-tetrafluoro-4-hydroxy-4-methoxybutanoate (169 ppm in total relative to the compound (2-2)), methyl 2,2,3,3-tetrafluoro-4-oxobutanate (30 ppm relative to the compound (2-2)), and water (75 ppm relative to the compound (2-2)). The results are shown in Table 1.

### Example 5: production of 2,2,3,3-tetrafluorobutane-1,4-diol

An experiment was performed as in Example 1 except that t-BuOH (1.0 g), which was used in Example 1, was changed to isopropyl alcohol (IPA) (1.0 g). Thereby, a composition was obtained containing the compound (2-2): 2,2,3,3-tetrafluorobutane-1,4-diol (0.64 g, yield 85%), 2,2,3,3-tetrafluoro-1,4-dimethoxybutane-1,4-diol + methyl 2,2,3,3-tetrafluoro-4-hydroxy-4-methoxybutanoate (190 ppm in total relative to the compound (2-2)), methyl 2,2,3,3-tetrafluoro-4-oxobutanate (70 ppm relative to the compound (2-2)), and water (204 ppm relative to the compound (2-2)). The results are shown in Table 1.

### Example 6: production of 2,2,3,3-tetrafluorobutane-1,4-diol

An experiment was performed as in Example 1 except that t-BuOH (1.0 g), which was used in Example 1, was changed to n-butyl alcohol (n-BuOH) (1.0 g). Thereby, a composition was obtained containing the compound (2-2): 2,2,3,3-tetrafluorobutane-1,4-diol (0.53 g, yield 710), 2,2,3,3-tetrafluoro-1,4-dimethoxybutane-1,4-diol + methyl 2,2,3,3-tetrafluoro-4-hydroxy-4-methoxybutanoate (221 ppm in total relative to the compound (2-2)), methyl 2,2,3,3-tetrafluoro-4-oxobutanate (34 ppm relative to the compound (2-2)), and water (127 ppm relative to the compound (2-2)). The results are shown in Table 1.

### Example 7: production of 2,2,3,3-tetrafluorobutane-1,4-diol

An experiment was performed as in Example 2 except that t-BuOH (1.0 g), which was used in Example 2, was changed to t-BuOH (0.9 g) + water (0.1 g). Thereby, a composition was obtained containing the compound (2-2): 2,2,3,3-tetrafluorobutane-1,4-diol (0.56 g, yield 75%), 2,2,3,3-tetrafluoro-1,4-dimethoxybutane-1,4-diol + methyl 2,2,3,3-tetrafluoro-4-hydroxy-4-methoxybutanoate (70 ppm in total relative to the compound (2-2)), methyl 2,2,3,3-tetrafluoro-4-oxobutanate (12 ppm relative to the compound (2-2)), and water (246 ppm relative to the compound (2-2)). The results are shown in Table 1.

### Example 8: production of 2-fluoropropane-1,3-diol

A reactor was charged with sodium borohydride (0.42 g) and IPA (1.0 g), followed by dropwise addition of the compound (1-1): diethyl fluoromalonate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-1): 2-fluoropropane-1,3-diol (0.18 g, yield 34%), 1,3-dimethoxy-2-fluoropropane-1,3-diol + ethyl 3-ethoxy-2-fluoro-3-hydroxypropanoate (209 ppm in total relative to the compound (2-1)), ethyl 2-fluoro-3-oxopropanoate (98 ppm relative to the compound (2-1)), and water (125 ppm relative to the compound (2-1)). The results are shown in Table 1.

### Example 9: production of 2,2,3,3,4,4-hexafluoropentane-1,5-diol

A reactor was charged with sodium borohydride (0.28 g) and IPA (1.0 g), followed by dropwise addition of a compound (1-3): dimethyl hexafluoroglutarate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-3): 2,2,3,3,4,4-hexafluoropentane-1,5-diol (0.76 g, yield 97%), 2,2,3,3,4,4-hexafluoro-1,5-dimethoxybutane-1,5-diol + methyl 2,2,3,3,4,4-hexafluoro-5-hydroxy-5-methoxybutanoate (270 ppm in total relative to the compound (2-3)), methyl 2,2,3,3,4,4-hexafluoro-5-oxobutanate (98 ppm relative to the compound (2-3)), and water (176 ppm relative to the compound (2-3)). The results are shown in Table 1.

### Example 10: production of 2,2,3,3,4,4-hexafluoropentane-1,5-diol

A reactor was charged with sodium borohydride (0.26 g) and IPA (1.0 g), followed by dropwise addition of a compound (1-4): diethyl hexafluoroglutarate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-4): 2,2,3,3,4,4-hexafluoropentane-1,5-diol (0.62 g, yield 86%), 2,2,3,3,4,4-hexafluoro-1,5-dimethoxybutane-1,5-diol + methyl 2,2,3,3,4,4-hexafluoro-5-hydroxy-5-methoxybutanoate (131 ppm in total relative to the compound (2-4)), methyl 2,2,3,3,4,4-hexafluoro-5-oxobutanate (16 ppm relative to the compound (2-4)), and water (238 ppm relative to the compound (2-4)). The results are shown in Table 1.

### Example 11: production of 2,2,3,3,4,4,5,5-octafluorohexane-1,6-diol

A reactor was charged with sodium borohydride (0.24 g) and IPA (1.0 g), followed by dropwise addition of a compound (1-5): dimethyl octafluoroadipate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) for washing the extract and liquid separation. The resulting ethyl acetate solution was dried over magnesium sulfide and filtered. The resulting ethyl acetate solution was then concentrated, whereby a composition was obtained containing a compound (2-5): 2,2,3,3,4,4,5,5-octafluorohexane-1,6-diol (0.76 g, yield 93%), 2,2,3,3,4,4,5,5-octafluoro-1,6-dimethoxybutane-1,6-diol + methyl 2,2,3,3,4,4,5,5-octafluoro-6-hydroxy-6-methoxybutanoate (184 ppm in total relative to the compound (2-5)), methyl 2,2,3,3,4,4,5,5-octafluoro-6-oxobutanate (111 ppm relative to the compound (2-5)), and water (314 ppm relative to the compound (2-5)). The results are shown in Table 1.

### Example 12: production of 2,3,3,3-tetrafluoro-2-(trifluoroethyl)propan-1-ol

A reactor was charged with sodium borohydride (0.17 g) and IPA (1.0 g), followed by dropwise addition of a compound (1-6): methyl 2,3,3,3-tetrafluoro-2-(trifluoromethyl)propanoate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-6): 2,3,3,3-tetrafluoro-2-(trifluoroethyl)propan-1-ol (0.76 g, yield 87%), 2,3,3,3-tetrafluoro-1-methoxy-2-(trifluoromethyl)propan-1-ol (114 ppm relative to the compound (2-6)), 2,3,3,3-tetrafluoro-2-(trifluoromethyl)propanal (41 ppm relative to the compound (2-6)), and water (67 ppm relative to the compound (2-6)). The results are shown in Table 1.

### Example 13: production of 2,2,2-trifluoroethanol

A reactor was charged with sodium borohydride (0.19 g) and IPA (1.0 g), followed by dropwise addition of a compound (1-7): 2,2,2-trifluoroethyl trifluoroacetate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then analyzed by GC, which confirmed that a composition was obtained containing a compound (2-7): 2,2,2-trifluoroethanol (0.45 g, yield 88%), 2,2,2-trifluoro-1-(2,2,2-trifluoroethoxy)ethan-1-ol (286 ppm relative to the compound (2-7)), 2,2,2-trifluoroacetaldehyde (192 ppm relative to the compound (2-7)), and water (24 ppm relative to the compound (2-7)). The results are shown in Table 1.

### Example 14: production of 3-fluoro-4-hydroxybutan-2-one

A reactor was charged with sodium borohydride (0.19 g) and IPA (1.0 g), followed by dropwise addition of a compound (1-8): 2,2,2-trifluoroethyl 2-fluoro-3-oxobutanate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-8): 3-fluoro-4-hydroxybutan-2-one (0.30 g, yield 57%), 3-fluoro-4-hydroxy-4-(2,2,2-trifluoroethoxy)butan-2-one (267 ppm relative to the compound (2-8)), 2-fluoro-3-oxobutanal (176 ppm relative to the compound (2-8)), and water (84 ppm relative to the compound (2-8)). The results are shown in Table 1.

### Example 15: production of 3,3,3-trifluoro-2-(trifluoromethyl)propane-1,2-diol

A reactor was charged with sodium borohydride (0.12 g) and water (3.0 g), followed by dropwise addition of a compound (1-9): methyl 3,3,3-trifluoro-2-hydroxy-2-trifluoromethylpropanoate (1.0 g) at 30°C or lower. The components were stirred for three hours at room temperature (RT). After this 3-hour stirring, 1 N hydrochloric acid (9 mL) was added to control the pH to 3 or lower. The solution was combined with diisopropyl ether (2 g) for extraction. The aqueous layer obtained by the liquid separation was further subjected to extraction with diisopropyl ether (2 g × twice), followed by addition of distilled water (2 g) to the extract for washing, followed by liquid separation. The resulting diisopropyl ether solution was dried over magnesium sulfide and filtered. The resulting diisopropyl ether solution was then concentrated, whereby a composition was obtained containing a compound (2-9): 3,3,3-trifluoro-2-(trifluoromethyl)propane-1,2-diol (0.71 g, yield 810), 3,3,3-trifluoro-1-methoxy-2-trifluoromethylpropane-1,2-diol (167 ppm relative to the compound (2-9)), 3,3,3-trifluoro-2-hydroxy-2-trifluoromethyl propanal (119 ppm relative to the compound (2-9)), and water (260 ppm relative to the compound (2-9)). The results are shown in Table 1.

### Example 16: production of 3,3,3-trifluoro-2-(trifluoromethyl)propane-1,2-diol

A reactor was charged with sodium borohydride (0.14 g) and IPA (1.0 g), followed by dropwise addition of a compound (1-10): methyl 2,3,3,3-tetrafluoro-2-(1,1,2-trifluoroallyloxy)propanoate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-10): 2,3,3,3-tetrafluoro-2-(1,1,2-trifluoroallyloxy)propan-1-ol (0.70 g, yield 78%), 2,3,3,3-tetrafluoro-1-methoxy-2-(1,1,2-trifluoroallyloxy)propan-1-ol (76 ppm relative to the compound (2-10)), 2,3,3,3-tetrafluoro-2-(1,1,2-trifluoroallyloxy)propanal (211 ppm relative to the compound (2-10)), and water (309 ppm relative to the compound (2-10)). The results are shown in Table 1.

### Example 17: production of 2,3,3,3-tetrafluoro-2-(1,1,2,2-tetrafluoro-2-(1,2,2-trifluorovinyloxy)ethoxy)propan-1-ol

A reactor was charged with sodium borohydride (0.10 g) and IPA (1.0 g), followed by dropwise addition of a compound (1-11): methyl 2,3,3,3-tetrafluoro-2-(1,1,2,2-tetrafluoro-2-(1,2,2-trifluorovinyloxy)ethoxy)propanoate (1.0 g). The components were stirred for 0.5 hours under reflux conditions caused by the heat of reaction. After this 0.5-hour stirring, 1 N hydrochloric acid (9 mL) was added to control the pH to 3 or lower. The solution was combined with ethyl acetate (2 g) for extraction. The aqueous layer obtained by the liquid separation was further subjected to extraction with ethyl acetate (2 g × twice), followed by addition of distilled water (2 g) to the extract for washing, followed by liquid separation. The resulting ethyl acetate solution was dried over magnesium sulfide and filtered. The resulting ethyl acetate solution was then concentrated, whereby a composition was obtained containing a compound (2-11): 2,3,3,3-tetrafluoro-2-(1,1,2,2-tetrafluoro-2-(1,2,2-trifluorovinyloxy)ethoxy)propan-1-ol (0.72 g, yield 78%), 2,3,3,3-tetrafluoro-1-methoxy-2-(1,1,2,2-tetrafluoro-2-(1,2,2-trifluorovinyloxy)ethoxy)propan-1-ol (135 ppm relative to the compound (2-11)), 2,3,3,3-tetrafluoro-2-(1,1,2,2-tetrafluoro-2-(1,2,2-trifluorovinyloxy)ethoxy)propanal (14 ppm relative to the compound (2-11)), and water (93 ppm relative to the compound (2-11)). The results are shown in Table 1.

### Example 18: production of 2,2,3,3-tetrafluorobutane-1,4-diol

An experiment was performed as in Example 2 except that the scale was 100 times as large as that of Example 2 and the duration of stirring was changed to 1.5 hours. Thereby, a composition was obtained containing the compound (2-2): 2,2,3,3-tetrafluorobutane-1,4-diol (64.7 g, yield 87%), 2,2,3,3-tetrafluoro-1,4-dimethoxybutane-1,4-diol + methyl 2,2,3,3-tetrafluoro-4-hydroxy-4-methoxybutanoate (84 ppm in total relative to the compound (2-1)), methyl 2,2,3,3-tetrafluoro-4-oxobutanate (31 ppm relative to the compound (2-2)), a boron compound (2600 ppm of boron in the boron compound relative to the compound (2-2)), and water (89 ppm relative to the compound (2-2)). The results are shown in Table 1.

### Example 19: production of 3,3,3-trifluoro-2-(trifluoromethyl)propane-1,2-diol

An experiment was performed as in Example 15 except that the scale was 150 times as large as that of Example 15 and the duration of stirring was changed to 18.0 hours. Thereby, a composition was obtained containing the compound (2-9): 3,3,3-trifluoro-2-(trifluoromethyl)propane-1,2-diol (112 g, yield 85%), 3,3,3-trifluoro-1-methoxy-2-trifluoromethylpropane-1,2-diol (19 ppm relative to the compound (2-9)), 3,3,3-trifluoro-2-hydroxy-2-trifluoromethyl propanal (13 ppm relative to the compound (2-9)), and water (28 ppm relative to the compound (2-9)). The results are shown in Table 1.

### Example 20: production of 2,2,3,3-tetrafluorobutane-1,4-diol

An experiment was performed as in Example 1 except that sodium borohydride (0.35 g), which was used in Example 1, was changed to sodium cyanoborohydride (0.69 g). Thereby, a composition was obtained containing the compound (2-2): 2,2,3,3-tetrafluorobutane-1,4-diol (0.60 g, yield 81%), 2,2,3,3-tetrafluoro-1,4-dimethoxybutane-1,4-diol + methyl 2,2,3,3-tetrafluoro-4-hydroxy-4-methoxybutanoate (156 ppm in total relative to the compound (2-2)), methyl 2,2,3,3-tetrafluoro-4-oxobutanate (28 ppm relative to the compound (2-2)), and water (100 ppm relative to the compound (2-2)). The results are shown in Table 1.

### Example 21: production of 2,2,3,3-tetrafluorobutane-1,4-diol

An experiment was performed as in Example 1 except that sodium borohydride (0.35 g), which was used in Example 1, was changed to sodium triacetoxyborohydride (2.33 g). Thereby, a composition was obtained containing the compound (2-2): 2,2,3,3-tetrafluorobutane-1,4-diol (0.52 g, yield 70%), 2,2,3,3-tetrafluoro-1,4-dimethoxybutane-1,4-diol + methyl 2,2,3,3-tetrafluoro-4-hydroxy-4-methoxybutanoate (207 ppm in total relative to the compound (2-2)), methyl 2,2,3,3-tetrafluoro-4-oxobutanate (52 ppm relative to the compound (2-2)), and water (163 ppm relative to the compound (2-2)). The results are shown in Table 1.

**[Table 1]**

| No. | Compound (1) | | Reducing agent | | | Solvent 1 | | Solvent 2 | | Total solvents | Ester concentration | Reaction temperature | Duration of reaction | Compound (2) | | |
|---|---|---|---|---|---|---|---|---|---|---|---|---|---|---|---|---|
| | Compound No. | [g] | Type | [g] | [eq. vs ester group] | Type | Amount used [g] | Type | Amount used [g] | Sum [g] | [mmol/g] | [°c] | [hr] | Compound No. | Amount obtained [g] | Yield [%] |
| Comparative Example 1 | 1-1 | 1.0 | NaBH₄ | 0.42 | 1.00 | MeOH | 1.0 | | | 1.0 | 5.6 | rfx. | 0.5 | 2-1 | 0.00 | 0 |
| Example 1 | 1-2 | 1.0 | NaBH₄ | 0.35 | 1.00 | t-BuOH | 1.0 | | | 1.0 | 4.6 | rfx. | 0.5 | 2-2 | 0.65 | 87 |
| Example 2 | 1-2 | 1.0 | NaBH₄ | 0.35 | 1.00 | t-BuOH | 1.0 | | | 1.0 | 4.6 | < 50 | 0.5 | 2-2 | 0.64 | 86 |
| Example 3 | 1-2 | 1.0 | NaBH₄ | 0.35 | 1.00 | t-BuOH | 1.5 | | | 1.5 | 3.1 | < 50 | 0.5 | 2-2 | 0.63 | 85 |
| Example 4 | 1-2 | 1.0 | NaBH₄ | 0.35 | 1.00 | t-BuOH | 3.0 | | | 3.0 | 1.5 | < 50 | 0.5 | 2-2 | 0.64 | 86 |
| Example 5 | 1-2 | 1.0 | NaBH₄ | 0.35 | 1.00 | IPA | 1.0 | | | 1.0 | 4.6 | rfx. | 0.5 | 2-2 | 0.64 | 85 |
| Example 6 | 1-2 | 1.0 | NaBH₄ | 0.35 | 1.00 | n-BuOH | 1.0 | | | 1.0 | 4.6 | rfx. | 0.5 | 2-2 | 0.53 | 71 |
| Example 7 | 1-2 | 1.0 | NaBH₄ | 0.35 | 1.00 | t-BuOH | 0.9 | Water | 0.1 | 1.0 | 4.6 | < 50 | 0.5 | 2-2 | 0.56 | 75 |
| Example 8 | 1-1 | 1.0 | NaBH₄ | 0.42 | 1.00 | IPA | 1.0 | | | 1.0 | 5.6 | rfx. | 0.5 | 2-1 | 0.18 | 34 |
| Example 9 | 13 | 1.0 | NaBH₄ | 0.28 | 1.00 | IPA | 1.0 | | | 1.0 | 3.7 | rfx. | 0.5 | 23 | 0.76 | 97 |
| Example 10 | 1-4 | 1.0 | NaBH₄ | 0.26 | 1.00 | IPA | 1.0 | | | 1.0 | 3.4 | rfx. | 0.5 | 2-4 | 0.62 | 86 |
| Example 11 | 1-5 | 1.0 | NaBH₄ | 0.24 | 1.00 | IPA | 1.0 | | | 1.0 | 3.1 | rfx. | 0.5 | 2-5 | 0.76 | 93 |
| Example 12 | 1-6 | 1.0 | NaBH₄ | 0.17 | 1.00 | IPA | 1.0 | | | 1.0 | 4.4 | rfx. | 0.5 | 2-6 | 0.76 | 87 |
| Example 13 | 1-7 | 1.0 | NaBH₄ | 0.19 | 1.00 | IPA | 1.0 | | | 1.0 | 5.1 | rfx. | 0.5 | 2-7 | 0.45 | 88 |
| Example 14 | 1-8 | 1.0 | NaBH₄ | 0.19 | 1.00 | IPA | 1.0 | | | 1.0 | 4.9 | rfx. | 0.5 | 2-8 | 0.30 | 57 |
| Example 15 | 1-9 | 1.0 | NaBH₄ | 0.12 | 0.72 | Water | 3.0 | | | 3.0 | 1.5 | RT | 3.0 | 2-9 | 0.71 | 81 |
| Example 16 | 1-10 | 1.0 | NaBH₄ | 0.14 | 1.00 | IPA | 1.0 | | | 1.0 | 3.7 | rfx. | 0.5 | 2-10 | 0.70 | 78 |
| Example 17 | 1-11 | 1.0 | NaBH₄ | 0.10 | 1.00 | IPA | 1.0 | | | 1.0 | 2.7 | rfx. | 0.5 | 2-11 | 0.72 | 78 |
| Example 18 | 1-2 | 100 | NaBH₄ | 34.70 | 1.00 | t-BuOH | 100 | | | 100 | 4.6 | < 50 | 1.5 | 2-2 | 64.7 | 87 |
| Example 19 | 1-9 | 150 | NaBH₄ | 18.07 | 0.72 | Water | 450.0 | | | 450.0 | 1.5 | RT | 18.0 | 2-9 | 112 | 85 |
| Example 20 | 1-2 | 1.0 | NaBH₃CN | 0.69 | 1.20 | t-BuOH | 1.0 | | | 1.0 | 4.6 | rfx. | 0.5 | 2-2 | 0.60 | 81 |
| Example 21 | 1-2 | 1.0 | NaBH(OAc)₃ | 2.33 | 1.20 | t-BuOH | 1.0 | | | 1.0 | 4.6 | rfx. | 0.5 | 2-2 | 0.52 | 70 |

## Claims

1. An alcohol production method comprising:
a step (1) of reacting a compound (1) and a metal hydride compound under conditions where at least one solvent selected from the group consisting of water and a C3 or higher protic solvent is present and the compound (1) is at a concentration of 1.0 mmol/g (amount of substance of compound (1)/total solvent amount) or higher to provide a compound (2),
the compound (1) being represented by the following formula (1): 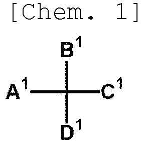 wherein
A¹ to D¹ are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (A): 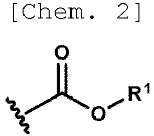
wherein R¹ is hydrogen or a C1-C10 hydrocarbon group in which at least one hydrogen atom is optionally replaced by a fluorine atom and which optionally contains a cyclic structure, a multiple bond, a carbonyl group, or an ether bond in a structure, or
a substituent represented by the following formula (B): 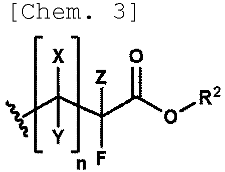
wherein
n is 0 to 5;
X, Y, and Z are each independently
hydrogen,
fluorine, or
a C1-C10 hydrocarbon group in which at least one hydrogen atom is optionally replaced by a fluorine atom and which optionally contains a cyclic structure, a multiple bond, a carbonyl group, or an ether bond in a structure; and
R² is hydrogen or a C1-C10 hydrocarbon group in which at least one hydrogen atom is optionally replaced by a fluorine atom and which optionally contains a cyclic structure, a multiple bond, a carbonyl group, or an ether bond in a structure,
not all of A¹ to D¹ are substituents represented by the formula (A),
at least one selected from A¹ to D¹ is a substituent represented by the formula (A) or the formula (B),
in the case where one to three selected from A¹ to D¹ are substituents represented by the formula (A), one or more of remaining substituents are fluorine, and
in the case where two or three selected from A¹ to D¹ are substituents each represented by the formula (A) or the formula (B), R¹ and R² are optionally bonded to each other at one or more carbon atoms each at any position,
the compound (2) being represented by the following formula (2): 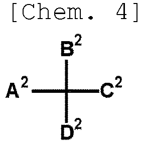 wherein
A² to D² are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (C) : 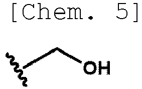 , or
a substituent represented by the following formula (D) : 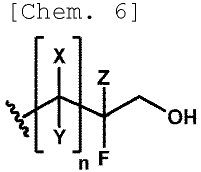
wherein
n is 0 to 5; and
X, Y, and Z are each independently
hydrogen,
fluorine, or
a C1-C10 hydrocarbon group in which at least one hydrogen atom is optionally replaced by a fluorine atom and which optionally contains a cyclic structure, a multiple bond, a carbonyl group, or an ether bond in a structure,
not all of A² to D² are substituents represented by the formula (C),
at least one selected from A² to D² is a substituent represented by the formula (C) or the formula (D), and
in the case where one to three selected from A² to D² are substituents represented by the formula (C), one or more of remaining substituents are fluorine.

2. The production method according to claim 1,
wherein the C3 or higher protic solvent includes at least one selected from the group consisting of n-propyl alcohol, isopropyl alcohol, n-butyl alcohol, sec-butyl alcohol, isobutyl alcohol, tert-butyl alcohol, n-pentyl alcohol, sec-pentyl alcohol, isopentyl alcohol, and neopentyl alcohol.

3. The production method according to claim 1 or 2,
wherein the metal hydride compound includes at least one selected from the group consisting of a boron hydride compound and an aluminum hydride compound.

4. The production method according to any one of claims 1 to 3,
wherein the metal hydride compound includes at least one selected from the group consisting of lithium borohydride, lithium aminoborohydride, lithium triethylborohydride, lithium tri(sec-butyl)borohydride, potassium borohydride, potassium tri(sec-butyl)borohydride, calcium borohydride, sodium borohydride, sodium cyanoborohydride, sodium triacetoxyborohydride, sodium bis(2-methoxyethoxy)aluminum hydride, zinc borohydride, nickel borohydride, and diborane.

5. The production method according to any one of claims 1 to 4,
wherein the metal hydride compound includes at least one selected from the group consisting of sodium borohydride, sodium cyanoborohydride, and sodium triacetoxyborohydride.

6. The production method according to any one of claims 1 to 5,
wherein the metal hydride compound is used in an amount of 1.2 equivalents or less relative to the ester group in the compound (1).

7. The production method according to any one of claims 1 to 6,
wherein X, Y, and Z are each independently hydrogen, fluorine, or a C1-C5 hydrocarbon group in which at least one hydrogen atom is optionally replaced by a fluorine atom.

8. The production method according to any one of claims 1 to 7,
wherein n is 0 to 3 and X, Y, and Z are each independently hydrogen or fluorine.

9. The production method according to any one of claims 1 to 8,
wherein R¹ or R² is a methyl group, an ethyl group, a n-propyl group, an isopropyl group, a n-butyl group, a sec-butyl group, an isobutyl group, a tert-butyl group, a n-pentyl group, a sec-pentyl group, an isopentyl group, a neopentyl group, a hexyl group, a 2-methylpentyl group, or a heptyl group in which at least one hydrogen atom is optionally replaced by a fluorine atom and which optionally contains an ether bond in a structure.

10. The production method according to any one of claims 1 to 9, further comprising:
a purifying step (2) including: adding an acid to a reaction solution containing the compound (2) obtained in the step (1) to achieve a pH of 3 or lower; performing extraction on the reaction solution to provide an extract; and washing the extract with water to remove a metal compound; or
a purifying step (3) including: adding an acid to a reaction solution containing the compound (2) obtained in the step (1) to achieve a pH of 3 or lower; and washing a solution at a lower layer with water to remove a metal compound.

11. A composition comprising:
a compound (2);
a metal compound; and
at least one selected from the group consisting of a compound (3) and a compound (4),
the compound (2) being represented by the following formula (2): 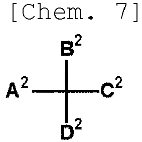 wherein
A² to D² are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (C) : 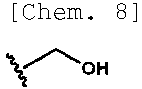 , or
a substituent represented by the following formula (D) : 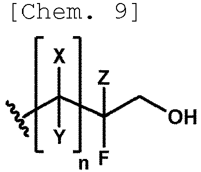
wherein
n is 0 to 5; and
X, Y, and Z are each independently
hydrogen,
fluorine, or
a C1-C10 hydrocarbon group in which at least one hydrogen atom is optionally replaced by a fluorine atom and which optionally contains a cyclic structure, a multiple bond, a carbonyl group, or an ether bond in a structure,
not all of A² to D² are substituents represented by the formula (C),
at least one selected from A² to D² is a substituent represented by the formula (C) or the formula (D), and
in the case where one to three selected from A² to D² are substituents represented by the formula (C), one or more of remaining substituents are fluorine,
the compound (3) being represented by the following formula (3): 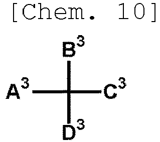 wherein
A³ to D³ are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (E) : 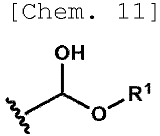
wherein R¹ is hydrogen or a C1-C10 hydrocarbon group in which at least one hydrogen atom is optionally replaced by a fluorine atom and which optionally contains a cyclic structure, a multiple bond, a carbonyl group, or an ether bond in a structure, or
a substituent represented by the following formula (F): 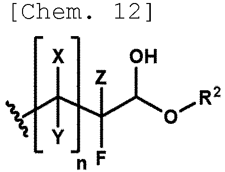
wherein
n is 0 to 5;
X, Y, and Z are each independently
hydrogen,
fluorine, or
a C1-C10 hydrocarbon group in which at least one hydrogen atom is optionally replaced by a fluorine atom and which optionally contains a cyclic structure, a multiple bond, a carbonyl group, or an ether bond in a structure; and
R² is hydrogen or a C1-C10 hydrocarbon group in which at least one hydrogen atom is optionally replaced by a fluorine atom and which optionally contains a cyclic structure, a multiple bond, a carbonyl group, or an ether bond in a structure,
not all of A³ to D³ are substituents represented by the formula (E),
at least one selected from A³ to D³ is a substituent represented by the formula (E) or the formula (F),
in the case where one to three selected from A³ to D³ are substituents represented by the formula (E), one or more of remaining substituents are fluorine, and
in the case where two or three selected from A³ to D³ are substituents each represented by the formula (E) or the formula (F), R¹ and R² are optionally bonded to each other at one or more carbon atoms each at any position,
the compound (4) being represented by the following formula (4): 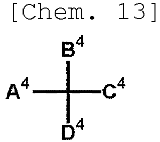 wherein
A⁴ to D⁴ are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (G) : 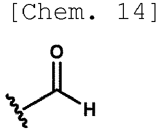 , or
a substituent represented by the following formula (H) : 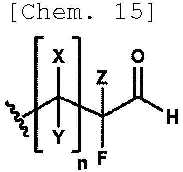
wherein
n is 0 to 5; and
X, Y, and Z are each independently
hydrogen,
fluorine, or
a C1-C10 hydrocarbon group in which at least one hydrogen atom is optionally replaced by a fluorine atom and which optionally contains a cyclic structure, a multiple bond, a carbonyl group, or an ether bond in a structure,
not all of A⁴ to D⁴ are substituents represented by the formula (G),
at least one selected from A⁴ to D⁴ is a substituent represented by the formula (G) or the formula (H), and
in the case where one to three selected from A⁴ to D⁴ are substituents represented by the formula (G), one or more of remaining substituents are fluorine.

12. The composition according to claim 11,
wherein the metal compound contains a metal element in an amount of 10000 ppm or less relative to the compound (2) .

13. The composition according to claim 11 or 12,
wherein the metal compound contains a metal element in an amount of 3000 ppm or less relative to the compound (2) .

14. The composition according to any one of claims 11 to 13,
wherein the compound (3) is contained in an amount of 10000 ppm or less relative to the compound (2).

15. The composition according to any one of claims 11 to 14,
wherein the compound (3) is contained in an amount of 1000 ppm or less relative to the compound (2).

16. The composition according to any one of claims 11 to 15,
wherein the compound (3) is contained in an amount of 300 ppm or less relative to the compound (2).

17. The composition according to any one of claims 11 to 16,
wherein the compound (4) is contained in an amount of 10000 ppm or less relative to the compound (2).

18. The composition according to any one of claims 11 to 17,
wherein the compound (4) is contained in an amount of 1000 ppm or less relative to the compound (2).

19. The composition according to any one of claims 11 to 18,
wherein the compound (4) is contained in an amount of 300 ppm or less relative to the compound (2).

20. A composition comprising water and a compound (2),
the water being contained in an amount of 1500 ppm or less relative to the compound (2),
the compound (2) being represented by the following formula (2): 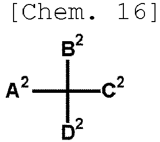 wherein
A² to D² are each independently
hydrogen,
fluorine,
a hydroxy group,
a C1-C10 hydrocarbon group in which at least one hydrogen atom is optionally replaced by a fluorine atom and which optionally contains a cyclic structure, a multiple bond, a carbonyl group, or an ether bond in a structure,
a substituent represented by the following formula (C) : 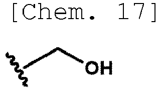 , or
a substituent represented by the following formula (D) : 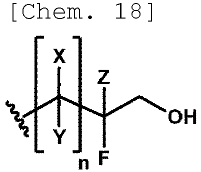
wherein
n is 0 to 5; and
X, Y, and Z are each independently
hydrogen,
fluorine, or
a C1-C10 hydrocarbon group in which at least one hydrogen atom is optionally replaced by a fluorine atom and which optionally contains a cyclic structure, a multiple bond, a carbonyl group, or an ether bond in a structure,
not all of A² to D² are substituents represented by the formula (C),
at least one selected from A² to D² is a substituent represented by the formula (C) or the formula (D), and
in the case where one to three selected from A² to D² are substituents represented by the formula (C), one or more of remaining substituents are fluorine.

21. The composition according to claim 20,
wherein the water is contained in an amount of 500 ppm or less relative to the compound (2).

22. The composition according to claim 20 or 21,
wherein the water is contained in an amount of 100 ppm or less relative to the compound (2).
